(19) Europäisches Patentamt
European Patent Office
Office européen des brevets

(11) EP 3 943 598 A1

(12) EUROPEAN PATENT APPLICATION

(43) Date of publication:
**26.01.2022 Bulletin 2022/04**

(21) Application number: **20186971.6**

(22) Date of filing: **21.07.2020**

(51) International Patent Classification (IPC):
***C12N 9/04*** *(2006.01)* ***C12N 9/02*** *(2006.01)*
***C12P 3/00*** *(2006.01)* ***C12P 7/02*** *(2006.01)*
***C12P 7/24*** *(2006.01)*

(52) Cooperative Patent Classification (CPC):
**C12Y 101/03009; C12N 9/001; C12P 3/00; C12P 7/24; C12Y 101/03007; C12Y 101/03013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Institut national de recherche pour l'agriculture l'alimentation et l'environnement 75007 Paris (FR)**
- **Centre National de la Recherche Scientifique 75016 Paris (FR)**
- **Université d'Aix Marseille 13007 Marseille (FR)**
- **Ecole Centrale de Marseille 13013 Marseille (FR)**

(72) Inventors:
- **BISSARO, Bastien 13008 Marseille (FR)**
- **BERRIN, Jean-Guy 13360 Roquevaire (FR)**
- **LAFOND, Michael 83220 Le Pradet (FR)**

(74) Representative: **Grosset-Fournier, Chantal Catherine et al Grosset-Fournier & Demachy 54, rue Saint-Lazare 75009 Paris (FR)**

(54) **USE OF UV-ACTIVATED ENZYMES TO IMPLEMENT OXIDATION REACTIONS AND THE CORRESPONDING PROCESSES**

(57) The present invention relates to the use of UV-activated Copper Radical Oxidase (CRO) enzymes in the implementation of oxidation reactions. The present invention also relates to a process for oxidizing organic compounds using enzymes which are activated by UV light.

The process according to the present invention also leads to concomitant formation of hydrogen peroxide, that can optionally be used in hydrogen peroxide mediated processes.

The present invention in particular relates to the oxidation of alcohols in aldehydes.

EP 3 943 598 A1

## Description

The invention relates to the use of UV-activated enzymes to implement oxidation reactions of organic compounds. The present invention also relates to a process for oxidizing organic compounds using enzymes which are activated by UV light.

The development of environmentally friendly processes in the chemical industry is of ongoing concern. In this respect, the use of enzymes in the production of chemicals has been explored in the past. One such class of enzymes are Copper Radical oxidases (CROs), that can catalyze a wide variety of oxidation reactions. These enzymes comprise a Copper ion in their active site, hence their name. Most CRO-enzymes belong to the CAZy family AA5 (Auxiliary Activity n° 5 family), which encompasses CAZy subfamily 1 (AA5_1) and CAZy subfamily 2 (AA5_2).

These CRO enzymes however need to be activated prior, or during the intended use in catalysis. Such activation is typically achieved by adding a redox-activator such as Horseradish peroxidase (HRP) to the reaction mixture comprising the enzyme. Horseradish peroxidase is an expensive and relatively unstable material, that, in addition, must be removed from the reaction mixture during the purification of the reaction product.

Hence the need for activating these enzymes using different methods, in particular methods that do not imply adding an exogeneous redox-activator.

Thus, one aim of the present invention is the use of UV-activated enzymes in the oxidation of an organic compound.

Another aim of the present invention is the activation of specific enzymes using UV light.

Yet another aim of the present invention is to provide a process for the oxidation of chemical compounds, which can be controlled, without the use of exogeneous redox-activators.

Yet another aim of the present invention is to provide control over said process.

Yet another aim of the present invention is the use of UV-activated enzymes in the production of hydrogen peroxide.

Yet another aim of the present invention is the use of the hydrogen peroxide thus produced.

Thus, a **first object** of the **present invention** concerns the use of UV-activated Copper Radical Oxidase (CRO) enzymes for the implementation of a chemical oxidation process of an organic compound.

The inventors have surprisingly found that Copper Radical Oxidase enzymes can be activated by UV light. This feature has not been described in the literature to date and allows for the use of these enzymes in oxidation reaction, without the need to add external redox activators.

Without being bound to theory, the mechanism of the UV-activation of CRO enzymes implies the formation of a radical on a Tyrosine residue that is located close to the copper that is present in the active site of the enzyme.

This was corroborated by the inventors in an EPR (Electron Paramagnetic Resonance) experiment, the results of which are shown in **Figure 1.**

Irradiation of the enzyme with UV light shows the appearance of 2 signals **(Figure 1B)**, as compared to the non-irradiated enzyme **(Figure 1A)**. These appearing signals are characteristic of radicals located in the proximity of the copper ion.

CRO-enzymes can exist in a *"mature"* form, and in a *"non-mature"* form. Non-mature enzymes are inactive towards the catalytic oxidation reactions according to the present invention.

For the UV-activation to happen, the enzyme should first be *"matured",* implying the copper mediated formation of a thioether bond between a cysteine residue and a Tyrosine residue in the enzymes active site (Ito et al., Novel Thioether Bond Revealed by a 1.7 A Crystal Structure of Galactose Oxidase, Nature 1991 Mar 7;350(6313):87-90, and Firbank et al., Crystal structure of the precursor of galactose oxidase: An unusual self-processing enzyme, Proc Natl Acad Sci USA. 2001 Nov 6; 98(23): 12932-12937).

In most cases, this maturation step occurs naturally during the production of the enzyme, and the isolated CRO enzyme is already mature. However, in rare cases, it is possible to achieve maturation *in vitro.* For instance, when enzyme production is carried out under metal-free conditions. The result is a precursor of the CRO-enzyme that does not have copper in the active site. This isolated precursor enzyme can subsequently be treated with a copper source, allowing for *in vitro* maturation (Roger et al., J. Am. Chem. Soc. 2000, 122, 5, 990-991).

**Scheme 1** shows the postulated catalytic cycle of the oxidation of a compound using a CRO enzyme.

A CRO enzyme in inactive form (semi-reduced), is exposed to UV light, thereby forming a CRO enzyme in active form (fully oxidized). This active species is able to oxidize a compound into an oxidized product, resulting in the concomitant formation of a fully reduced CRO enzyme. Said fully reduced enzyme subsequently reduces oxygen into hydrogen peroxide, thus regenerating the fully oxidized active form that can be involved in another reaction cycle. Turnover numbers of more than 10,000 can thus be achieved.

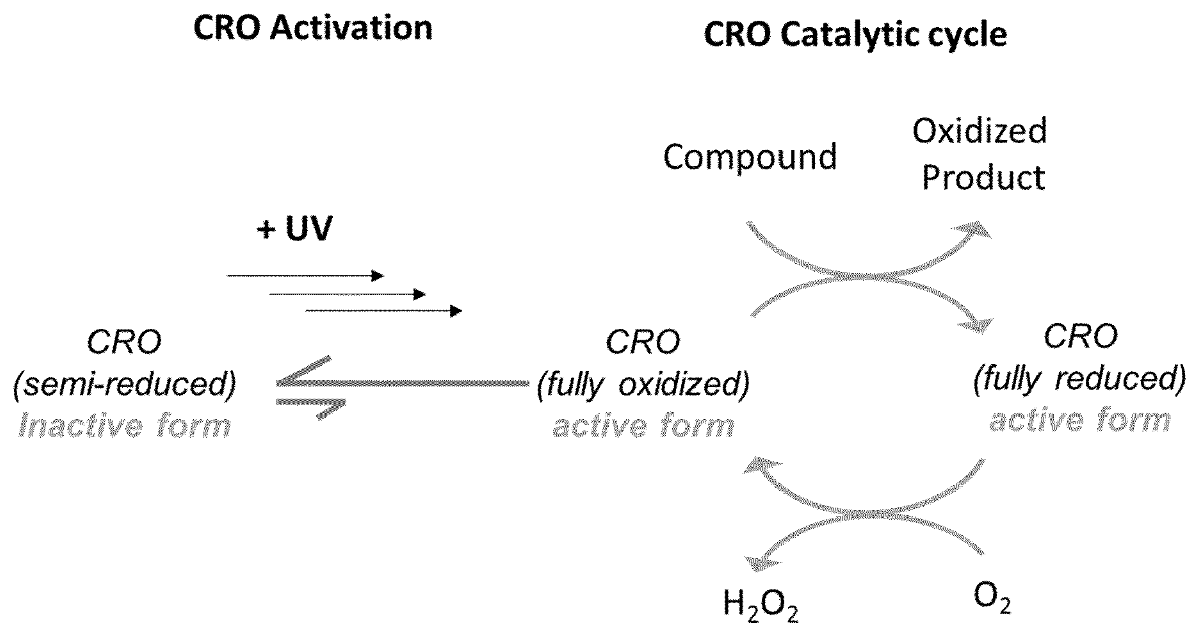


**Scheme 1** Catalytic cycle of a reaction implying UV-activation of a CRO enzyme

According to another embodiment, the present invention concerns the use of UV-activated Copper Radical Oxidase (CRO) enzymes as described above, wherein the chemical oxidation of the organic compound is followed by the formation of hydrogen peroxide.

In a preferred embodiment of the present invention; the formation of hydrogen peroxide is concomitant.

The present invention also concerns the use of UV-activated Copper Radical Oxidase (CRO) enzymes for the implementation of a chemical oxidation process of an organic compound, in particular, wherein the chemical oxidation of the organic compound is followed by the formation of hydrogen peroxide.

**A second object of the present invention** concerns a process for the chemical oxidation of an organic compound,

said process comprising the step of contacting an organic compound bearing an oxidizable function with at least one Copper-Radical Oxidase (CRO) enzyme in the presence of molecular oxygen,

wherein said at least one CRO enzyme is activated by a step of exposing said at least one CRO enzyme to UV light, to obtain a UV-activated CRO enzyme,

whereby the organic compound is oxidized into an oxidized organic product,

and whereby hydrogen peroxide is generated.

According to the present invention, the expression *"oxidizable function"* relates to a functional group able to be oxidized, in particular a carbon-heteroatom single bond, that is oxidized into a carbon heteroatom double bond, wherein the heteroatom preferably chosen from O, N, or S, the oxidizable function is in particular a -C-OH group that is oxidized into a -C=O group.

According to the present invention, a step of contacting an organic compound with at least one CRO-enzyme, refers to bringing together said organic compound with said at least one CRO-enzyme. The organic compound thus is "in contact" with the at least one CRO-enzyme, and thus becomes available to the said at least one CRO-enzyme, allowing them to interact.

The organic compound and the at least one CRO-enzyme are typically brought in solution together, in the same reaction flask.

According to the present Invention, the expression "at least one CRO-enzyme" refers to one or more than one CRO-enzymes of different sequence, in particular from 1 to 3 CRO enzyme(s), more in particular 1, 2 or 3 CRO-enzyme(s). For example, in a step of contacting an organic compound with 2 CRO-enzymes, a mixture of galactose oxidase and an alcohol oxidase, or a mixture of 2 galactose oxidases of different sequence, is used.

According to the present invention, *"UV light"* is a form of electromagnetic radiation having a wavelength ranging from 200 to 400 nm.

With "200 to 400 nm" is also meant the following ranges: 200 to 350 nm, 200 to 300 nm, 200 to 250 nm, 200 to 225 nm, 240 to 400 nm, 300 to 400 nm, 350 to 400 nm, 225 to 375 nm, 240 to 350 nm, , 240 to 320 nm, and 270 to 290 nm.

Below 200 nm harmful species can be generated, resulting in side-reactions.

According to the present invention, the expression *"exposing said at least one CRO enzyme to UV light"* indicates that the CRO-enzyme is irradiated with UV light, whereby the UV light reaches the enzyme. In this respect, an experimental setup can be a UV-lamp that is immersed into the solution comprising the enzyme. Alternatively, the UV-source can be placed outside the reactor that contains the solution comprising the enzyme, said reactor being made of a material that does not block the UV light, such as Quartz. The UV-source can be artificial, such as a lamp, or natural, such as sun-light.
The exposure of the at least one CRO enzyme to UV light, during the step of exposing said at least on CRO enzyme with UV light, can occur at different stages of the process.
Thus, according to a preferred embodiment, the present invention relates to a process for chemical oxidation as previously described, wherein the step of exposing said at least one CRO enzyme to UV light is carried out during the step of contacting.
Thus, according to another preferred embodiment, the present invention concerns a process for chemical oxidation as previously described, wherein the step of exposing said at least one CRO enzyme to UV light is carried out before the step of contacting.
In this embodiment, the at least one CRO enzyme is exposed to UV light before the step of contacting with the organic compound to be oxidized. In other word, the enzyme is pre-exposed leading to a pre-activated enzyme.
Said pre-exposing the enzyme to UV light is preferably carried out by solubilizing the enzyme in a reaction solvent and providing UV light. Once activation is achieved, which can be analyzed using an EPR analysis, the enzyme is contacted with the organic compound.
Thus, according to another preferred embodiment, the present invention concerns a process for chemical oxidation as previously described, wherein the step of exposing said at least one CRO enzyme to UV light is carried out both before and during the step of contacting.
In this embodiment, the enzyme is pre-exposed to UV light, and is also exposed to UV light during the contacting step.
According to another preferred embodiment, the present invention concerns a process for chemical oxidation as previously described, wherein the step of exposing said at least one CRO enzyme to UV light is carried out

- during the step of contacting,
- before the step of contacting, or
- both before and during the step of contacting,

in particular wherein said step of exposing to UV light before the step of contacting is maintained for 1 second to about 10 minutes, followed by contacting said UV-activated enzyme with said organic compound.
According to another preferred embodiment, the present invention concerns a process for chemical oxidation as described above,
wherein during the contact of said organic compound with said enzyme, the exposure to UV light is continuous or intermittent, preferably intermittent.
In some embodiments, the exposure to UV light is continuous, meaning uninterrupted exposure to UV light, whereby the exposure is maintained during the entire course of the reaction.
In other embodiments, the exposure to UV light is intermittent, whereby said exposure is interrupted by one or more periods of non-exposure. For example, by switching the light source on and off.
As shown in the postulated reaction mechanism of Scheme 1, the fully oxidized active form can be converted into a semi-reduced form, leading to an inactive enzyme that should be re-exposed to UV light in order to be activated. Thus, by continuously exposing the enzyme to UV light during the oxidation reaction, the enzyme is continuously regenerated *in situ.*
On the contrary, by interrupting the exposure to UV light, the enzyme can become semi-reduced and inactive, and a decrease in catalytic activity is observed.
Subsequent renewed UV light exposure leads to reactivation of the enzyme.
This feature of the invention allows for control over the reaction kinetics, in a sense by switching the enzyme activity on and off. This feature also allows, if desired, to perform reactions in a sequential manner.
According to another preferred embodiment, the present invention concerns a process for chemical oxidation as described above,
wherein during the contact of said organic compound with said enzyme, the exposure to UV light is intermittent,
and preferably is maintained for one or more periods of 1 second to 1 hour, in particular 1 second to 10 minutes, followed by one or more non-exposure periods of 1 second to 1 hour.
The exposure periods may be of equal length, or may be of different length than the non-exposure periods.
In case of more than one 1 exposure period, the subsequent exposure periods may be of equal length or may be of different length.
In case of more than one 1 non-exposure period, the subsequent non-exposure periods may be of equal length or may

be of different length.

By *"one or more periods"* should also be understood, 1 to 1000 periods, 1 to 500 periods; 1 to 250 periods, 1 to 100 periods, 1 to 10 periods, 10 to 1000 periods, 100 to 1000 periods, 250 to 1000 periods, 500 to 1000 periods, 10 to 500 periods, or 100 to 250 periods. These ranges include all individual integer values, so that for example "*1 to 10 periods"* has the meaning of 1, 2, 3, 4, 5, 6, 7, 8, 9, or 10 periods.

By "*1 second to 1 hour"* should be understood all individual time-values comprised within this range, so that for example "*1 second to 10 seconds"* includes 1, 2, 3, 4, 5, 6, 7, 8, 9, and 10 seconds.

By "*1 second to 1 hour"* should in particular be understood 1 second to 45 minutes, 1 second to 30 minutes, 1 second to 15 minutes, 1 second to 10 minutes, 1 second to 5 minutes, 1 second to 2 minutes, 1 to 60 seconds, 1 to 50 seconds, 1 to 40 seconds, 1 to 30 seconds, 1 to 20 seconds, 1 to 10 seconds, 1 to 5 seconds, 1 minute to 1 hour, 10 minutes to 1 hour, 30 minutes to 1 hour, 1 minute to 30 minutes, or 5 minutes.

According to another preferred embodiment, the present invention concerns a process for chemical oxidation as described above,

wherein during the contact of said organic compound with said enzyme, the exposure to UV light is continuous or intermittent,

in particular, wherein during the contact of said organic compound with said enzyme, the exposure to UV light is intermittent, and preferably is maintained for one or more periods of 1 second to 1 hour, followed by one or more non-exposure periods of 1 second to 1 hour.

According to another preferred embodiment, the present invention concerns a process for chemical oxidation as described above,

wherein the UV light has a wavelength comprised from 240 to 320 nm, preferably from 270 to 290 nm, more preferably has a wavelength of about 280 nm.

According to another preferred embodiment, the present invention concerns a process for chemical oxidation as described above,

wherein, during the step of exposing said at least one CRO enzyme to UV light, the enzyme is exposed to UV light having a light intensity comprised from 0.01 to 1000 $mW/cm^2$, in particular from 1 to 100 $mW/cm^2$.

According to the present invention, it should be understood that the light intensity values correspond to the light intensity of the UV light to which the enzyme is exposed, before activation of said enzyme in other words, of the UV light that reaches the reaction medium. Said light intensity can be different from the light intensity of the UV light emitted from the UV-source.

According to another preferred embodiment, the present invention concerns a process for chemical oxidation as described above,

wherein oxygen is constantly or discontinuously provided during the contact of said organic compound with said enzyme.

When oxygen is constantly provided, the provision of oxygen is uninterrupted, whereby oxygen provision is maintained during the entire course of the reaction.

Discontinuous provision of oxygen means that periods of oxygen provision are altered with periods of non-provision of oxygen. The oxygen provision is thus interrupted by one or more periods of non-provision. For example, by stopping the bubbling of oxygen into the reaction mixture.

As for the intermittent provision of UV light, this feature allows for control over the reaction kinetics, and allows, if desired, to perform reaction in a sequential manner.

The oxygen provision periods may be of equal length, or may be of different length than the non-provision periods. In case of more than one 1 oxygen provision period, the subsequent oxygen provision periods may be of equal length or may be of different length.

In case of more than one 1 non-provision period, the subsequent non-provision periods may be of equal length or may be of different length.

The provision of molecular oxygen into the reaction mixture can be carried out by using open reaction vessels that are exposed to ambient air. The provision of oxygen is preferably carried out by bubbling oxygen or air into the reaction mixture, wherein the above mentioned non-provision of oxygen can be achieved for example by bubbling an inert gas into the reaction mixture in place of the oxygen or air.

Alternatively, and in another embodiment of the Invention, the molecular oxygen is provided by the presence of a catalase enzyme in the reaction mixture, said catalase enzyme being able to convert hydrogen peroxide into molecular oxygen and water.

According to another preferred embodiment, the present invention concerns a process for chemical oxidation as described above,

wherein the step of exposing said at least one CRO enzyme to UV light is carried out before the step of contacting,

wherein said step of exposing to UV light before the step of contacting is maintained for 1 second to about 10 minutes, followed by contacting said UV-activated enzyme with said organic compound.

By "*1 second to 10 minutes"* should be understood all individual time-values comprised within this range, so that for

example "*1 second to 10 seconds*" includes 1, 2, 3, 4, 5, 6, 7, 8, 9, and 10 seconds.

By "*1 second to 10 minutes*" should in particular be understood 1 second to 5 minutes, 1 second to 1 minute, 1 to 30 seconds, 1 to 10 seconds, 10 seconds to 10 minutes, 30 seconds to 10 minutes, 1 to 10 minutes, 5 to 10 minutes, 30 seconds to 5 minutes, 1 to 5 minutes, 2 to 5 minutes, or 2 to 3 minutes.

According to another preferred embodiment, the present invention concerns a process for chemical oxidation as described above,

wherein said process comprises between the step exposing said at least one CRO enzyme to UV light before the step of contacting, and the step of contacting said UV-activated enzyme with said organic compound,

a step of transfer whereby the organic compound and the UV-activated enzyme are mixed, said step of transfer in particular being achieved in less than 10 minutes, preferably less than 1 minutes, and more preferably in less than 10 seconds.

With *"a step of transfer"* is meant the act of transferring the UV-activated enzyme into a solution comprising the organic substrate, or conversely transferring the organic substrate into the solution comprising the enzyme.

The transfer step is in particular carried out by transferring the UV-activated enzyme to the solution comprising the organic compound.

This transfer step should be performed quickly, preferably in less than 10 seconds, to prevent the UV-activated enzyme from deactivating in time.

In particular, the step of transfer should take place in a time sufficiently short to retain at least 80% of the initial activity of the UV-activated enzyme, preferably at least 90%, more preferably at least 95%.

In this respect the activity of the UV-activated enzyme is a parameter related to a batch of enzymes. The enzyme activity can be measured by methods known to the person skilled in the art.

According to another preferred embodiment, the present invention concerns a process for chemical oxidation as described above,

wherein said process is carried out in an aqueous medium, preferably in a buffered aqueous medium, more preferably at a temperature comprised between 20 and 50 °C preferably at a temperature of about 23 °C.

The aqueous buffer is in particular a sodium phosphate buffer, citrate-phosphate buffer, a tris-HCl buffer or HEPES, preferably a sodium phosphate buffer.

The buffered aqueous medium preferably has a pH ranging from 6 to 10, in particular from 7 to 9, more in particular 7 or 8.

According to another preferred embodiment, the present invention concerns a process for chemical oxidation as described above,

wherein the at least one CRO enzyme is of fungal origin.

According to another preferred embodiment, the present invention concerns a process for chemical oxidation as described above,

wherein the at least one CRO enzyme is of bacterial origin.

According to another preferred embodiment, the present invention concerns a process for chemical oxidation as described above,

wherein the at least one CRO enzyme belongs to the AA5 family.

Families and subfamilies of the enzymes used in the present enzymes are classified according to the CAZy database, which describes the families of structurally-related catalytic and carbohydrate-binding modules (or functional domains) of enzymes that degrade, modify, or create glycosidic bonds.

Alternatively, enzymes can be identified by an enzyme commission number, or EC-number :

| Enzyme | EC number |
|---|---|
| Galactose oxidase | 1.1.3.9 |
| Glyoxal oxidase | 1.2.3.15 |
| Alcohol oxidase | 1.1.3.13 |
| Aryl alcohol oxidase | 1.1.3.7 |

The at least one CRO enzyme in particular belongs to the AA5_1 or AA5_2 subfamilies.

According to another preferred embodiment, the present invention concerns a process for chemical oxidation as described above,

wherein the at least one CRO enzyme belongs to the AA5_2 subfamily and is an alcohol oxidase (AlcOx), preferably is an alcohol oxidase extracted from *Colletotrichum graminicola,* in particular having SEQ *ID* NO: 1, or having at least 60%, in particular at least 70% identity with SEQ *ID* NO: 1.

SEQ *ID* NO: 1 corresponds to the following sequence, wherein the signal peptide is highlighted in underlined,

and the catalytic domain is highlighted in **bold:**

MPTLRSALRNLPAALLALAAACEA**QNVGKWGPMVKFPVVPVAVALVPETGNLLV WSSGWPNRWTTAGNGKTYTSLYNVNTGNISDAIVQNTQHDMFCPGTSLDADGR IIVTGGSSAAKTSVLDFKKGESSPWTPLSNMQISRGYQSSCTTSEGKIFVIGGSFSG AGTRNGEVYDPKANTWTKLAGCPVKPLVMQRGMFPDSHAWLWSWKNGSVLQ AGPSKKMNWYDTKGTGSNTPAGLRGTDEDSMCGVSVMYDAVAGKIFTYGGGK GYTGYDSTSNAHILTLGEPGQAVQVQKLANGKYNRGFANAVVMPDGKIWVVG GMQKMWLFSDTTPQLTPELFDPATGSFTPTTPHTVPRNYHSTALLMADATIWSG GGGLCGANCKENHFDGQFWSPPYLFEADGVTPAKRPVIQSLSDTAVRAGAPITIT MQDAGAYTFSMIRVSATTHTVNTDQRRIPLDGQDGGDGKSFTVNVPNDYGVAIP GYYMLFAMNEAGVPCVAQFFKVTL**

According to the present Invention, with the expression "at least 70% identity" should also be understood: at least 71%, at least 72%, at least 73%, at least 74%, at least 75%, at least 76%, at least 77%, at least 78%, at least 79%, at least 80%at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99%.

According to another preferred embodiment, the present invention concerns a process for chemical oxidation as described above,

wherein the at least one CRO enzyme belongs to the AA5_2 subfamily and is a galactose oxidase (GalOx) and more preferably is a galactose oxidase extracted from *Fusarium graminearum,* in particular having SEQ *ID* NO: 2, or having at least 60%, in particular at least 70% identity with SEQ *ID* NO: 2.

SEQ *ID* NO:2 corresponds to the following sequence, wherein the signal peptide is highlighted in underlined, and the catalytic domain is highlighted in **bold:**

MKHLLTLALCFSSINAVAVTVPHKAVGTGIPEGSLQFLSLRASAPIGSAISRNNWAVT CDSAQSGNECNKAIDGNKDTFWHTFYGANGDPKPPHTYTIDMKTTQNVNGLSMLPR QDGNQNGWIGRHEVYLSSDGTNWGSPVASGSWFADSTTKYSNFETRPARYVRLVAI TEANGQPWTSIAEINVFQASSYTAP**QPGLGRWGPTIDLPIVPAAAAIEPTSGRVLM WSSYRNDAFGGSPGGITLTSSWDPSTGIVSDRTVTVTKHDMFCPGISMDGNGQIV VTGGNDAKKTSLYDSSSDSWIPGPDMQVARGYQSSATMSDGRVFTIGGSWSGGV FEKNGEVYSPSSKTWTSLPNAKVNPMLTADKQGLYRSDNHAWLFGWKKGSVF QAGPSTAMNWYYTSGSGDVKSAGKRQSNRGVAPDAMCGNAVMYDAVKGKILT FGGSPDYQDSDATTNAHIITLGEPGTSPNTVFASNGLYFARTFHTSVVLPDGSTFI TGGQRRGIPFEDSTPVFTPEIYVPEQDTFYKQNPNSIVRVYHSISLLLPDGRVFNG GGGLCGDCTTNHFDAQIFTPNYLYNSNGNLATRPKITRTSTQSVKVGGRITISTD SSISKASLIRYGTATHTVNTDQRRIPLTLTNNGGNSYSFQVPSDSGVALPGYWML FVMNSAGVPSVASTIRVTQ**

According to another preferred embodiment, the present invention concerns a process for chemical oxidation as described above,

wherein the at least one CRO enzyme belongs to the AA5_2 subfamily and is an aryl alcohol oxidase (AAO) and is preferably extracted from *Colletotrichum graminicola,* in particular having SEQ *ID* NO: 3, or having at least 60%, in particular at least 70% identity with SEQ *ID* NO: 3.

SEQ *ID* NO:3 corresponds to the following sequence, wherein the signal peptide is highlighted in underlined, and the catalytic domain is highlighted in **bold:**

MVRSCAYKAIAAASLLSQLASAAITSCPNNETVWETPIGVKYTLCPGSDYQNGGASL
QTVRDIQSSLECAKICDSDARCNRAVYDNVNKACDVKNSTNPMQWAADDRFETIRL
TNDLPEGAFISTCSFNETSYRVPETNAEYRICPDTDYTGVNAKVVEGVTTIQACAELC
SNTQDCRKSVFDHINNACAIKAAEPATSIFWVQDKQFSTIRLPENID**PAVKGKWGDLI
RLPVIPVAAYIVPSYPEPSRLLFFSSWSNDAFSGASGMTQFGDYDFATGAISQRTV
TNTHHDMFCPGISQLEDGRILIQGGSDADTVSIYDPATNEFTRGPNMTLARGYQT
SCTLSNGKVFTIGGAYSGERVGKNGEVYDPVANAWTYLPGADFRPMLTNDHEG
IWREDNHAWLFGWKNGSIFQAGPSKDQHWYGIQGNGTVAKAATRDDDDAMC
GVWVMYDAVAGKIFSAGGSPDYTDSPATQRAHITTIGEPNTPAEVERVADMGFP
RGFANAVVLPDGQVLVTGGQRMSLVFTNTDGILVAELFNPETREWKQMAPMA
VPRNYHSVSILLPDATVFSGGGGMCWVQNVGDSTAGCDKTVDHSDGEIFEPPYL
FNEDGSRAARPVISAISADPIKAGATLTFTVEGVEGQGTAALIRLGSVTHSVNSDQ
RRVPLNVTVSGNEYSATLPDDYGILLPGYYYLFVSTPQGTPSIAKTVHVIL**

According to another preferred embodiment, the present invention concerns a process for chemical oxidation as described above,
wherein the at least one CRO enzyme belongs to the AA5_1 subfamily and is a glyoxal oxidase (GLOx) and more preferably is a glyoxal oxidase extracted from *Pycnoporus cinnabarinus,* in particular having SEQ *ID* NO: 4, or having at least 60%, in particular at least 70% identity with SEQ *ID* NO: 4.

SEQ *ID* NO:4 corresponds to the following sequence, wherein the signal peptide is highlighted in underlined, and the catalytic domain is highlighted in bold:

MFQTTLHLLFVLVVTGRGLA**APSTPTGWQFNLKAERSGIVALESIVVSPTLVVFFD
RATNDPLQINNHSAWGALWNLETSTVRALDVLTNSFCASGALLSNGTMASIGGD
PNGFPGNPAIHPGTQAIRLFEPCDSPTGEGCTLFEDPVTLHLLEKRWYPSSVRIFD
GSLLIVGGMHEETPFYNTDPALSFEFFPPKESTPRPSEFLNRSLPANLFPRVFALP
DGKVFMVANNQSIIYDIEANTERILPDIPNNVRVTNPIDGSAILLPLSPPDFVPEVL
VCGGTQTDTIDPSLLTSQTPASSQCSRIRLDEEGIARGWEVEHMLEGRMMPELV
HLPNGQVLIANGARTGFAAIASVSDPVGGSNADHAVLVPSLYTPDAPLGTRISNV
GLPSSGIARVYHSSITLTPQGNFLIAGSNPNNNSSVTAGVKFPSEFRVQTLDPPFM
FVERPKILSMPKKLAFGKSFTVPIAVPSTLAHPGAKVQVSLMDLGFSSHAFHSSA
RLVFMNAKISQDGKSLTFTTPPNGRVYPPGPATIFLTIDDVTSEGAWVMMGSGN
PPPTLE**

According to another preferred embodiment, the present invention concerns a process for chemical oxidation as described above,
wherein the at least one CRO enzyme is a GlxA-type enzyme which is preferably extracted from the bacterium *Streptomyces lividans,* in particular having SEQ *ID* NO: 5, or having at least 60%, in particular at least 70% identity with SEQ *ID* NO: 5.

GlxA enzymes are bacterial homologues of fungal CAZy family AA5, in particular of galactose oxidases.

SEQ *ID* NO:5 corresponds to the following sequence, wherein the signal peptide is highlighted in underlined, and the catalytic domain is highlighted in **bold:**

MKDRAGRRRARRFAIGTAVVVALAGMNGPWLYRFSTE**KYHQYKINQPEYKAANG**
**KWEIIEFPEKYRQNTIHAALLRTGKVLMVAGSGNNQDNSDDKQYDTRIWDPVK**
**GTIKKVPTPSDLFCTGHTQLANGNLLIAGGTKRYEKLKGDVTKAGGLMVVHNE**
**NPDKPITLPAGTKFTGKENGKTFVSKDPVLVPRAEKVFDPATGAFVRNDPGLGRI**
**YVEAQKSGSAYETGTEDNYRVQGLSGADARNTYGIAQKLALDKKDFQGIRDAF**
**EFDPVAEKYIKVDPMHEARWYPTLTTLGDGKLSVSGLDDIGQLVPGKNEVYDP**
**KTKAWTYTDKVRQFPTYPALFLMQNGKIFYSGANAGYGPDDVGRTPGVWDVE**
**TNKFTKVPGMSDANMLETANTVLLPPAQDEKYMVIGGGGVGESKLSSEKTRIA**
**DLKADDPKFVDGPSLEKGTRYPQASILPDDSVLVSGGSQDYRGRGDSNILQARLY**
**HPDTNEFERVADPLVGRNYHSGSILLPDGRLMFFGSDSLYADKANTKPGKFEQRI**
**EIYTPPYLYRDSRPDLSGGPQTIARGGSGTFTSRAASTVKKVRLIRPSASTHVTDV**
**DQRSIALDFKADGDKLTVTVPSGKNLVQSGWYMMFVTDGEGTPSKAEWVRVP**

According to another preferred embodiment, the present invention concerns a process for chemical oxidation as described above,
wherein the at least one CRO enzyme belongs to the AA5_2 subfamily and is an alcohol oxidase (AlcOx), preferably is an alcohol oxidase extracted from *Colletotrichum graminicola,* in particular having SEQ *ID* NO: 1, or having at least 60%, in particular at least 70% identity with SEQ *ID* NO: 1,
or wherein the at least one CRO enzyme belongs to the AA5_2 subfamily and is a galactose oxidase (GalOx) and more preferably is a galactose oxidase extracted from *Fusarium graminearum,* in particular having SEQ *ID* NO: 2, or having at least 60%, in particular at least 70% identity with SEQ *ID* NO: 2.
or wherein the at least one CRO enzyme belongs to the AA5_2 subfamily and is an aryl alcohol oxidase (AAO) and is preferably extracted from *Colletotrichum graminicola,* in particular having SEQ *ID* NO: 3, or having at least 60%, in particular at least 70% identity with SEQ *ID* NO: 3.
or wherein the at least one CRO enzyme belongs to the AA5_1 subfamily and is a glyoxal oxidase (GLOx) and more preferably is a glyoxal oxidase extracted from *Pycnoporus cinnabarinus,* in particular having SEQ *ID* NO: 4, or having at least 60%, in particular at least 70% identity with SEQ *ID* NO: 4.
or wherein the at least one CRO enzyme is a GlxA-type enzyme which is preferably extracted from the bacterium *Streptomyces lividans,* in particular having SEQ *ID* NO: 5, or having at least 60%, in particular at least 70% identity with SEQ *ID* NO: 5.

According to another preferred embodiment, the present invention concerns a process for chemical oxidation as described above, wherein said organic compound is chosen from:

- saturated ($C_1$ to $C_{20}$) primary alcohols,
- unsaturated ($C_1$ to $C_{20}$) primary alcohols,
- saturated ($C_1$ to $C_{20}$) secondary alcohols,
- unsaturated ($C_1$ to $C_{20}$) secondary alcohols,
- ($C_3$ to $C_{10}$) cyclic alcohols,
- aryl alcohols,
- heteroaryl alcohols, and
- geminal diols.

According to another preferred embodiment, the present invention concerns a process for chemical oxidation as described above, wherein said organic compound is chosen from:

- saturated ($C_1$ to $C_{20}$) primary alcohols,
- unsaturated ($C_1$ to $C_{20}$) primary alcohols,
- aryl alcohols comprising a primary hydroxyl group,
- heteroaryl alcohols comprising a primary hydroxyl group, and
- geminal diols.

According to another preferred embodiment, the present invention concerns a process for chemical oxidation as described above, wherein said organic compound is chosen from:

- saturated ($C_1$ to $C_{20}$) primary alcohols,

- allylic alcohols,
- aryl alcohols comprising a primary hydroxyl group linked to the aryl group by a $C_1$ alkyl group, and
- geminal diols.

The alcohols according to the present invention can also comprise more than one hydroxyl groups. Examples of such polyols are 1,2-propanediol, 1,3-propanediol, 1,4-propanediol, glycerol, sorbitol, xylitol,
or carbohydrates, in particular carbohydrates chosen from the group consisting of sugars such as galactose, lactose, melibiose, raffinose, glucose, xylose, arabinose, ribose, fructose, mannose, sucrose, lactose, cellobiose and xyloglucan,
or macromolecules, in particular natural polymers comprising aliphatic chains bearing hydroxyl groups, such plant cutins.

According to the present invention, primary alcohols are oxidized into aldehydes, secondary alcohols are oxidized into ketones, and gem-diols are oxidized into carboxylic acids.

The invention in particular concerns primary alcohols being oxidized into aldehydes and gem-diols being oxidized into carboxylic acids.

The term *"saturated (C1 to C20) primary alcohols"* means a primary alcohol comprising a saturated alkyl chain comprising 1 to 20 carbon atoms, linear or branched, in particular comprising 1, 2, 3, 4, 5, 6, 7, 8, 9, 10, 11, 12, 13, 14, 15, 16, 17, 18, 19, or 20 carbon atoms, said saturated primary alcohols being for example chosen from methanol, ethanol, 1-propanol, 1-butanol, 1-pentanol, 1-hexanol, 1-heptanol, (±)-2-methyl-1-butanol and (S)-(-)-2-methyl-1-butanol,
in particular methanol being oxidized to formaldehyde, or 1-hexanol being oxidized to 1-hexanal.

The term *"unsaturated (C1 to C20) primary alcohols"* means a primary alcohol comprising an alkyl chain comprising 1 to 20 carbon atoms, linear or branched, in particular comprising 1, 2, 3, 4, 5, 6, 7, 8, 9, 10, 11, 12, 13, 14, 15, 16, 17, 18, 19, or 20 carbon atoms, said alkyl chain further comprising one or more double, or triple bonds,
in particular allylic alcohols,
said unsaturated primary alcohols being for example chosen from cis-3-hexen-1-ol, 2,4-hexadiene-1-ol, retinol and geraniol.

The term *"saturated (C1 to C20) secondary alcohols"* means a secondary alcohol comprising a saturated alkyl chain comprising 1 to 20 carbon atoms, linear or branched, in particular comprising 1, 2, 3, 4, 5, 6, 7, 8, 9, 10, 11, 12, 13, 14, 15, 16, 17, 18, 19, or 20 carbon atoms, said saturated secondary alcohols being for example chosen from 2-propanol, 2-butanol.

The term *"unsaturated (C1 to C20) secondary alcohols*" means a secondary alcohol comprising an alkyl chain comprising 1 to 20 carbon atoms, linear or branched, in particular comprising 1, 2, 3, 4, 5, 6, 7, 8, 9, 10, 11, 12, 13, 14, 15, 16, 17, 18, 19, or 20 carbon atoms, said alkyl chain further comprising one or more double, or triple bonds,

The term *"($C_3$ to $C_{10}$) cyclic alcohols"* means a cyclic compound comprising from 3 to 10 carbon atoms, in particular comprising 3, 4, 5, 6, 7, 8, 9 or 10 carbon atoms,
said cycle can be saturated or unsaturated,
said cycle can further comprise a heteroatom, in particular chosen from O, N, or S,
said cycle comprising at least one hydroxyl group, said cyclic alcohols being for example chosen from cyclopentanol; 2-cyclopentenol, cyclohexanol and 2-cyclohexenol,

The term *"aryl alcohols"* means a primary or secondary alcohol further comprising at least one aryl group, said at least one aryl group being linked to the hydroxyl group, in particular a primary hydroxyl group, by a ($C_1$ to $C_{20}$) linear or branched alkyl chain as previously defined, in particular by a $C_1$ alkyl chain,
said aryl alcohols being in particular aryl alcohols comprising a primary hydroxyl group linked to the aryl group by a $C_1$ alkyl group, or aryl alcohols comprising an allylic alcohol attached to the aryl group,
said aryl alcohols being for example chosen from benzyl alcohol, 4-methoxy benzyl alcohol, 2,3-dimethoxy benzyl alcohol, 4-hydroxy benzyl alcohol, 2-phenyl ethanol, cinnamyl alcohol, coniferyl alcohol, sinapyl alcohol,
in particular benzyl alcohol being oxidized to benzaldehyde.

The term *"heteroaryl alcohols"* means an aryl alcohol as previously defined, wherein the aryl group further comprised a heteroatom chosen from O, N, or S,
said heteroaryl alcohols being for example chosen from furfuryl alcohol and p-coumaryl alcohol.

The term *"geminal diols"* means a compound of structure $R_1HC(OH)_2$, being oxidized into $R_1CO_2H$. wherein $R_1$ represents

- a ($C_1$ to $C_{20}$) alkyl group, linear or branched,
- a ($C_3$ to $C_{10}$) cyclic alkyl group,
- a carboxylic acid group,
- an aryl group, or heteroaryl.

In another embodiment, the present invention concerns a process for chemical oxidation as described above, wherein said organic compound is of natural origin, or is derived from a compound of natural origin.

Examples of such compounds are waxes, cutins, hemicellulose such as xyloglucan,

According to another preferred embodiment, the present invention concerns a process for chemical oxidation as described above, wherein said organic compound is chosen from:

- saturated ($C_1$ to $C_{20}$) primary alcohols,
- unsaturated ($C_1$ to $C_{20}$) primary alcohols,
- saturated ($C_1$ to $C_{20}$) secondary alcohols,
- unsaturated ($C_1$ to $C_{20}$) secondary alcohols,
- ($C_3$ to $C_{10}$) cyclic alcohols,
- aryl alcohols,
- heteroaryl alcohols, and
- geminal diols,

in particular chosen from:

- saturated ($C_1$ to $C_{20}$) primary alcohols,
- allylic alcohols,
- aryl alcohols comprising a primary hydroxyl group linked to the aryl group by a $C_1$ alkyl group, and
- geminal diols.

According to another preferred embodiment, the present invention concerns a process for chemical oxidation as described above,

wherein said enzyme is an alcohol oxidase (AlcOx), and wherein said organic compound is a primary alcohol, or an aryl alcohol, and the obtained oxidized organic product is an aldehyde.

According to another preferred embodiment, the present invention concerns a process for chemical oxidation as described above, wherein said enzyme is an alcohol oxidase (AlcOx), and wherein said organic compound is an aryl alcohol comprising a primary hydroxyl group linked to the aryl group by a $C_1$ alkyl group, in particular selected from benzyl alcohol, 4-nitrobenzyl alcohol, anisyl alcohol, veratryl alcohol and 4-hydroxybenzyl alcohol,

or aryl alcohols comprising an allylic alcohol attached to the aryl group, in particular cinnamyl alcohol.

According to another preferred embodiment, the present invention concerns a process for chemical oxidation as described above, wherein said enzyme is an alcohol oxidase (AlcOx), and wherein said organic compound is a saturated, or unsaturated ($C_1$ to $C_{20}$) primary alcohol, linear or branched, in particular chosen from n-butanol, n-pentanol, n-hexanol or 2,4-hexadiene-1-ol.

According to another preferred embodiment, the present invention concerns a process for chemical oxidation as described above, wherein said enzyme is an alcohol oxidase (AlcOx), and wherein said organic compound is a naturally-occurring polymer comprising long aliphatic chains bearing hydroxyl functions or a sugar, in particular polymers chosen from waxes, cutins and hemicellulose.

According to another preferred embodiment, the present invention concerns a process for chemical oxidation as described above, wherein said enzyme is an alcohol oxidase (AlcOx), and wherein said organic compound is :

- a primary alcohol and the obtained oxidized organic product is an aldehyde,
- an aryl alcohol comprising a primary hydroxyl group linked to the aryl group by a C1 alkyl group, in particular selected from benzyl alcohol, 4-nitrobenzyl alcohol, anisyl alcohol, veratryl alcohol and 4-hydroxybenzyl alcohol, or aryl alcohols comprising an allylic alcohol attached to the aryl group, in particular cinnamyl alcohol,
- a saturated, or unsaturated (C1 to C20) primary alcohol, linear or branched, in particular chosen from n-butanol, n-pentanol, n-hexanol or 2,4-hexadiene-1-ol, or
- a naturally-occurring polymer comprising long aliphatic chains bearing hydroxyl functions or a sugar, in particular polymers chosen from waxes, cutins and hemicellulose.

According to another preferred embodiment, the present invention concerns a process for chemical oxidation as described above, wherein said enzyme is a glyoxal oxidase (GLOx), and wherein said organic compound is :

- 5-hydroxymethylfurfuryl alcohol, or
- lignocellulose derived compounds, in particular glyoxal, methyl glyoxal, glyoxylic acid, formaldehyde or glycerol.

Said glyoxal, methyl glyoxal, glyoxylic acid and formaldehyde being in particular in the form of a hemi-acetal.

According to another preferred embodiment, the present invention concerns a process for chemical oxidation as described above,

wherein said enzyme is a galactose oxidase (GalOx), and wherein said organic compound is:

- forest and agricultural biomass, in particular fibres, and hemicelluloses, in particular compounds comprising a galactopyranose moiety, more in particular xyloglucan.

**A third object of the present invention** is a process for the production of hydrogen peroxide, said process comprising the step of contacting one organic compound bearing an oxidizable function with at least one Copper-Radical Oxidase (CRO) enzyme in the presence of molecular oxygen, under exposure to UV light,
whereby hydrogen peroxide is generated.

**A fourth object of the present invention** is the use of the hydrogen peroxide obtained by a process for the production of hydrogen peroxide as previously described, or by a process for chemical oxidation as described above, in hydrogen peroxide-driven enzymatic reactions.

According to another preferred embodiment, the present invention concerns the use of hydrogen peroxide as previously described, wherein the hydrogen peroxide-driven enzymatic reaction is chosen from decarboxylations, hydroxylations, halogenations, epoxidations, sulfoxidations, Baeyer-Villiger oxidations.

According to another preferred embodiment, the present invention concerns the use of hydrogen peroxide as previously described, wherein the hydrogen peroxide-driven enzymatic reaction consists in the enzymatic conversion of said hydrogen peroxide into oxygen and water, in particular using a catalase enzyme.

In this embodiment, the hydrogen peroxide that is formed from molecular oxygen during the CRO-catalyzed oxidation reaction is re-converted into molecular oxygen. Thus, regeneration of molecular oxygen occurs, allowing for the CRO-catalyzed oxidation reaction to continue to take place.

According to another preferred embodiment, the present invention concerns the use of hydrogen peroxide as previously described, wherein the hydrogen peroxide-driven enzymatic reactions consists in the degradation of a polysaccharide, said reaction comprising contacting said polysaccharide with one or more lytic polysaccharide monooxygenase (LPMO), in the presence of an external source of electrons,
said source of electrons being in particular a reducing agent.
Examples of reducing agents are organic compounds such as ascorbic acid or cysteine, photocatalysts such as Titanium dioxide, or enzymes such as cellobiose dehydrogenase.

According to another preferred embodiment, the present invention concerns the use of hydrogen peroxide as previously described,
wherein the hydrogen peroxide-driven enzymatic reaction is chosen from decarboxylations, hydroxylations, halogenations, epoxidations, sulfoxidations and Baeyer-Villiger oxidations,
or wherein the hydrogen peroxide-driven enzymatic reaction consists in the enzymatic conversion of said hydrogen peroxide into oxygen and water, in particular using a catalase enzyme,
or wherein the hydrogen peroxide-driven enzymatic reactions consists in the degradation of a polysaccharide, said reaction comprising contacting said polysaccharide with one or more lytic polysaccharide monooxygenase (LPMO), in the presence of an external source of electrons, said source of electrons being in particular a reducing agent.

The following figures and examples illustrate the practice of the present invention in some of its embodiments, the figures and examples should not be construed as limiting the scope of the invention.

## FIGURES

**Figure 1** represents the EPR spectra showing the UV-activation of a CRO enzyme. **Figure 1A** represents a non-activated AA5_2 AlcOx enzyme, and **Figure 1B** the enzyme after UV activation. The arrows identify signals that appear after the UV-activation.

**Figure 2** represents the conversion of benzyl alcohol into benzaldehyde by an AlcOx CRO under different reaction conditions. The y-axis corresponds to the benzaldehyde concentration (μM) and the x-axis corresponds to the reaction time expressed in minutes.
▪ represents a reaction without activation of the enzyme (control reaction), ▲ represents a reaction wherein the enzyme is activated with horseradish peroxidase, • represents a reaction according to the Invention, the enzyme being pre-activated with UV light, and ♦ represents a reaction wherein the buffer is exposed to UV light, in the absence of enzyme (negative control)

**Figure 3** represents the conversion of benzyl alcohol into benzaldehyde by an AlcOx CRO under different reaction conditions. The y-axis corresponds to the benzaldehyde concentration (μM) and the x-axis corresponds to the reaction time expressed in minutes.
▪ represents a reaction in the dark, without UV light (control reaction), ▲ represents a reaction with intermittent UV

exposure, but in the absence of a CRO enzyme (negative control), • represents a reaction according to the Invention, wherein the enzyme was pre-activated by UV, and ♦ represents a reaction according to the Invention, wherein the enzyme intermittently activated by UV light, the grey vertical bars represent the periods of light exposure.

## EXEMPLES

### Abbreviations and definitions

| | |
|---|---|
| BMGY medium | Buffered Glycerol-complex Medium |
| BMMY medium | Buffered Methanol-complex. Medium |
| EPR | Electron Paramagnetic Resonance |
| HRP | Horseradish peroxidase |
| $OD_{600\ nm}$ | Optical Density measured at 600 nm |
| Pluronic E8100 | Non-ionic surfactant (anti-foam) |
| PTM1 trace salts | Salt-metals mix solution |
| rpm | Revolutions per minute |
| YPD agar | Yeast Extract Peptone Dextrose Agar |
| g | relative centrifugal force |

### General remarks

Most chemicals were purchased from Sigma-Aldrich (Darmstadt, Germany) or VWR.
HRP type II (MW 33.89 kDa) and catalase from bovine liver (monomer MW 62.5 kDa) were purchased from Sigma-Aldrich. Molar concentrations of HRP was estimated by Bradford assay.
All alcohol substrates stock solutions were prepared in $H_2O$ whenever possible, aliquoted and stored at -20 °C. The concentration of $H_2O_2$ stock solution was verified at 240 nm ($\varepsilon^{240}$ = 43.6 $M^{-1}.cm^{-1}$).

### EXAMPLE 1: DNA Cloning and strain production

The DNA cloning and strain production was performed according to methods described in the literature:
DNA cloning and strain production of the alcohol oxidase from *Colletotrichum graminicola* (CgrAlcOx, Genbank ID XM_008096275.1, Uniprot ID E3QHV8) was performed according to D. (Tyler) Yin et al., "Structure-function characterization reveals new catalytic diversity in the galactose oxidase and glyoxal oxidase family," Nat. Commun., vol. 6, p. 10197, Dec. 2015.

DNA cloning and strain production of the galactose oxidase from *Fusarium graminearum* (FgrGalOx, Genbank ID XM_011327027.1, Uniprot ID I1 S2N3) was performed according to O. Spadiut et al., "A comparative summary of expression systems for the recombinant production of galactose oxidase," Microb. Cell Fact., vol. 9, pp. 1-13, 2010.

DNA cloning and strain production of the glyoxal oxidase from *Pycnoporus cinnabarinus* (GLOx, *Pci*GLOx-2, ORF ID BN946_scf184747.g42, Uniprot ID A0A060SYB0) was performed according to M. Daou et al., "Heterologous production and characterization of two glyoxal oxidases from Pycnoporus cinnabarinus," Appl. Environ. Microbiol., vol. 82, no. 16, pp. 4867-4875, 2016.

DNA cloning and strain production of the aromatic alcohol oxidase from *Colletotrichum graminicola* (*CgrAAO,* Genbank ID EFQ27661, Uniprot ID E3Q9X3) was performed according to Y. Mathieu et al., "Discovery of a Fungal Copper Radical Oxidase with High Catalytic Efficiency toward 5-Hydroxymethylfurfural and Benzyl Alcohols for Bioprocessing," ACS Catal., vol. 10, no. 5, pp. 3042-3058, 2020.

### EXAMPLE 2: Heterologous enzyme production

For preliminary tests, all proteins were first produced in 2 L flasks. To this end, single colonies of *P. pastoris* X33 expressing the gene of interest were individually streaked on a YPD agar plate containing Zeocin (100 $\mu g.mL^{-1}$) and incubated 3 days at 30 °C. A single colony was then used to inoculate 10 mL of YPD, in a 50 mL sterile Falcon tube, incubated during 5 h (30 °C, 160 *rpm).* This pre-culture was used to inoculate at 0.2% (vol/vol), 500 mL of BMGY medium in a 2 L baffled flask, incubated during approximately 16 h (30 °C, 200 *rpm*) until the $OD_{600\ nm}$ reached 4-6. The produced cellular biomass was then harvested by centrifugation (5 min, 16 °C, 3,000 g). The cell pellet was resuspended in 100 mL BMMY medium in a 500 mL flask supplemented with $CuSO_4$ (500 $\mu$M) and methanol (1%, vol/vol) and incubated for 3 days (16 °C, 200 *rpm),* with daily additions of methanol (1% added, vol/vol). Then, the extracellular medium was

recovered by centrifugation (10 min, 4 °C, 3,000 g) and the supernatant filtered on 0.45 μm membrane (Millipore, Massachusetts, USA) and stored at 4 °C prior to purification.

*Cgr*AAO and *Pci*GLOx-2 were produced according to known procedures: Y. Mathieu et al., "Discovery of a Fungal Copper Radical Oxidase with High Catalytic Efficiency toward 5-Hydroxymethylfurfural and Benzyl Alcohols for Bioprocessing," ACS Catal., vol. 10, no. 5, pp. 3042-3058, 2020 and M. Daou et al., "Heterologous production and characterization of two glyoxal oxidases from Pycnoporus cinnabarinus," Appl. Environ. Microbiol., vol. 82, no. 16, pp. 4867-4875, 2016, respectively.

**EXAMPLE 3: Heterologous enzyme bioreactor production**

*Cgr*AlcOx and *Fgr*GalOx were also produced at larger scale, in bioreactors. Bioreactor production was carried out in a 1.3-L New Brunswick BioFlo 115 fermentor (Eppendorf, Germany). Precultures were prepared as described above for flask production and were used to inoculate at 0.2% 100 mL of BMGY medium, in a 500 mL flask, incubated (30 °C, 200 *rpm)* until the $OD_{600nm}$ reached 2-6. Four hundred mL of basal salt medium containing 1.8 mL PTM1 trace salts (both made according to the *P. pastoris* fermentation process guidelines - Invitrogen - version B 053002) were inoculated with 10 % (vol/vol) of the BMGY culture. Temperature was set to 30 °C. One hundred μL of Pluronic E8100 (BASF, Germany) were added after 6 h of culture to prevent foam. After full consumption of glycerol (as indicated by a return of dissolve oxygen (DO) level at 100 %), sorbitol-methanol transition phase was initiated by addition of 80 mL sorbitol (250 $g.L^{-1}$ stock solution), 1.6 mL PTM1 traces salts and 2 mL methanol. After full consumption of carbon sources, the temperature was lowered to 20 °C and a methanol fed-batch was initiated with a feeding rate of 3.9 mL/h/L (mL per hour per liter of initial fermentation volume) of a methanol solution complemented with PTM1 trace salts (12 mL/L). New additions of 100 μL Pluronic E8100 were made after 30 h and 53 h of fermentation. Methanol feeding rate was increased to 7.8 mL/h/L after 53 h of fermentation. Throughout the fermentation, pH was maintained at 5 by automated adjustment with $NH_3$ base. Air flow was maintained at 0.5 slpm (standard liter per minute). A cascade with a set point of 20% dissolved oxygen is maintained through agitation between 400 to 900 rpm and the percentage of pure oxygen addition between 0 to 50%. Fermentation was ceased after 118 hours. The harvested biomass was centrifuged (10 min, 5500 g, 4°C). The supernatant was filtered through a 0.45-μm membrane (Millipore), flash-frozen in liquid nitrogen and stored at -80 °C. Flash-freezing did not cause any enzyme activity loss, for both CgrAlcOx and FgrGalOx.

**EXAMPLE 4: Protein purification**

The filtered culture broth was buffer exchanged by ultrafiltration through a 10 kDa cut-off polyethersulfone membrane (Vivacell 250, Sartorius Stedim Biotech GmbH, Germany) with Tris-HCl (50 mM pH 8.7) or Tris-HCl (50 mM pH 8.0) for CRO-AlcOx and CRO-GalOx, respectively. CRO-AlcOx was purified by anion exchange chromatography by loading the crude protein sample on a DEAE-20 mL HiPrep FF 16/10 column (GE Healthcare, Illinois, USA), equilibrated with buffer A1 (Tris-HCl, 50 mM, pH 8.7) and connected to an Äkta Express system (GE Healthcare). Elution was performed by applying a linear gradient from 0 to 50% of buffer B1 (Tris-HCl, 50 mM, pH 8.7 + 1 M NaCl) over 15 column volumes (CV) at a flow rate of 3 $mL.min^{-1}$.

*Fgr*GalOx was purified by ionic metal affinity chromatography by loading the crude protein sample on a His-Trap HP 5-mL column (GE Healthcare, Buc, France) and connected to an Äkta Xpress system (GE Healthcare). Prior to loading, the column was equilibrated with buffer A2 (50 mM Tris-HCl, pH 7.8, 150 mM NaCl). After loading, non-specific proteins were eluted by applying a first washing step of 5 CV at 2% of buffer B2 (50 mM Tris-HCl, pH 7.8, 150 mM NaCl, 500 mM imidazole) and the target protein was eluted during a second step of 5 CV at 30% of buffer B2. Loading and elution were carried out at a flow rate of 3 $mL.min^{-1}$. In all cases, the collected fractions were analyzed by SDS-PAGE in 10% polyacrylamide precast gel (Bio-Rad) stained with Coomassie blue. Fractions containing the recombinant enzyme were pooled, concentrated and buffer exchanged in sodium phosphate (50 mM, pH 7.0) or sodium acetate (50 mM, pH 5.2), for *Cgr*AlcOx and *Fgr*GalOx, respectively. The protein concentration was determined by UV absorption at 280 nm using a Nanodrop ND-200 spectrophotometer (Thermo Fisher Scientific, Massachusetts, USA) for *Cgr*AlcOx ($\varepsilon$ = 101215 $M^{-1}.cm^{-1}$), *Fgr*GalOx ($\varepsilon$ = 124,135 $M^{-1}.cm^{-1}$), *Pci*GLOx-1 ($\varepsilon$ = 47,690 $M^{-1}.cm^{-1}$), *Cgr*AAO ($\varepsilon$ = 107,760 $M^{-1}.cm^{-1}$).

**EXAMPLE 5: Spectrophotometric monitoring of benzyl alcohol oxidation**

Benzyl alcohol can be oxidized by several CROs, including *Cgr*AlcOx, *Fgr*GalOx and *Cgr*AAO. The oxidation of benzyl alcohol into benzaldehyde was monitored by developing a simple absorbance assay consisting in measuring changes in absorbance at 254 nm upon oxidation of benzyl alcohol (1.5 mM) by the CRO (10 nM final concentration). Reactions were carried out in sodium-phosphate buffer (50 mM, pH 7.0), at 23 °C, in UV-transparent cuvettes (1 mL reaction volume). In positive controls (carried out in the dark), horseradish peroxidase (HRP, 50 $\mu g.mL^{-1}$) was added

to the mixture. The reactions were initiated by adding the CRO (100 μL of 100 nM stock solution), which was either stored in the dark or UV-exposed (*vide infra*). The reaction was mixed by vigorously pipetting up and down. In negative controls, the enzyme stock solution was replaced by sodium phosphate buffer (50 mM, pH 7.0), kept in the dark or UV-exposed. The absorbance (pathlength = 1 cm) was measured using an Evolution 201 UV-Vis spectrophotometer (Thermo-Fisher). Given a 1:1 stoichiometry of the reaction, one can calculate the concentration of benzaldehyde according to Equation 1, where the molecular extinction coefficient of benzaldehyde at 254 nm is 57-fold higher ($\varepsilon^{254}{}_{benzaldehyde}$ = 8,497 $M^{-1}.cm^{-1}$) than its alcohol counterpart ($\varepsilon^{254}{}_{BnOH}$ = 149 $M^{-1}.cm^{-1}$).

$$[\mathrm{Benzaldehyde}]_t = (\mathrm{Abs}^{254\ nm}{}_t - \mathrm{Abs}^{254\ nm}{}_{t0})/(\varepsilon^{254}{}_{benzaldehyde}\text{-}\varepsilon^{254}{}_{BnOH}) \qquad \text{(equation 1)}$$

**EXAMPLE 6: CRO pre-photoactivation**

Pre-activation experiments consisted in exposing the enzyme stock solution (100 nM, 1 mL) to UV light during a given amount of time (usually 10 min) before transferring (ca. 30 sec step) a fraction (100 μL) to the reaction mixture. The reaction was then monitored as described above. In preliminary tests, a UV-lamp model EF-180/F (Spectroline, Spectronics Corporation, Westbury, USA) was used, delivering 254 nm UV light at a maximum power of 1350 $\mu W/cm^2$, equivalent to 29 μmoles of photons.$s^{-1}.m^{-2}$), positioned side-wise (relatively to the reaction cuvette) at a 3 cm distance from the cuvette.
**Figure 2** shows the reaction kinetics observed for the benzaldehyde formation using an AlcOx CRO enzyme.

EXAMPLE **7: CRO intermittent illumination**

In intermittent illumination experiments, the full reaction mixture, i.e. containing buffer, CRO and benzyl alcohol, was submitted to cycles of UV exposure (2 min) followed by on-line spectrophotometric monitoring (2 min). In such experiments, the illumination set-up was the same as described in example 6.
**Figure 3** shows the reaction kinetics observed for the benzaldehyde formation using an AlcOx CRO enzyme, an applying intermittent UV light exposure.

**EXAMPLE 8: Electron paramagnetic resonance**

*Cgr*AlcOx (50 μM), prepared in sodium phosphate buffer (50 mM, pH 7.0), was flash-frozen in liquid nitrogen and continuous wave EPR spectrum was recorded. Then, the sample was thawed, exposed to UV light (280 nm +/- 10 nm, sample positioned side wise and at 40 cm from lamp source, 400 mW received by the sample) during 10 min and flash-frozen again before recording a new EPR spectrum. Controls containing only buffer were also carried out. The illumination system was an Arc Lamp Housing equipped with a xenon-mercury bulb (Newport, USA, model 67005) and connected to an OPS-A150 Arc Lamp power supply (50-500W) (Newport, USA). The power was set to 350 W. EPR spectra were recorded on a Bruker Elexsys E500 spectrometer operating at X-band at 110 K (BVT 3000 digital temperature controller) with the following acquisition parameters: number of scans, 3; modulation frequency, 100 kHz; modulation amplitude, 5 G; attenuation, 10 dB and microwave power, 20 mW.

**EXAMPLE 9: Optimized small-scale photobiocatalytic reaction**

Optimal illumination conditions are determined by exposing the full reaction mixture to different light intensities (by varying the distance between the light source and the reaction vessel and/or varying the power supply) and different light wavelengths (by using different bandpass filters). Continuous *versus* intermittent light exposure is also probed, where the duration of intermittent light and "dark" time are varied (from 0 sec to 10 min). An optimal reaction condition is defined as a reaction yielding a stable reaction kinetic and/or a high final product yield (e.g., > 70%). The quantity of reaction products is measured off-line by sampling regularly the reaction mixture and stopping the reaction (by either adding EDTA (10 mM final) or acidifying the mixture with HCl (1N final)) prior to quantification.
Different CRO / substrate couples are tested, including:

- CRO-AlcOx/benzyl alcohol or fatty alcohols (e.g., hexanol)
- CRO-GalOx/galactose
- CRO-GLOx/glyoxal or methyl glyoxal or glyoxylic acid
- CRO-AAO/5-hydroxymethylfurfural (HMF)

In standard reaction conditions, the CRO (10 nM - 1 μM) is mixed with the substrate (3-30 mM) in buffered

aqueous medium (sodium phosphate (50 mM, pH 8.0) for CRO-AlcOx and CRO-GalOx, sodium 2,2'-dimethylsuccinate (50 mM, pH 6.0) for CRO-GLOx, sodium phosphate (50 mM, pH 7.0) for CRO-AAO). The reaction is carried out at 23 °C, under magnetic stirring, in Quartz cuvettes (Hellma, France). The effect of the supply of $O_2$ is also tested by bubbling through a syringe either air or a mixture of $N_2/O_2$ (various ratio of $O_2$ between 0 and 100% v/v), with a gas flow rate of 100 mL/min.

The illumination system is an Arc Lamp Housing equipped with a xenon-mercury bulb (Newport, USA, model 67005) and connected to an OPS-A150 Arc Lamp power supply (50-500W) (Newport, USA). The power is set to 350 W. A bandpass filter (280 ± 10 nm) with 50 mm optical diameter (Edmund Optics, Lyon) is mounted on the lamp. The light intensity received by the sample is measured outside of the reaction vessel, on the front side facing the light beam, with an optical power meter (Newport, USA).

**EXAMPLE 10: Photobiocatalytic reactions coupled to a secondary enzymatic system**

Using optimized illumination conditions, we evaluate the effect of secondary enzymatic reactions on the primary photobiocatalytic CRO reaction. A first secondary reaction is the conversion of $H_2O_2$ produced *in situ* into $H_2O$ and $O_2$ by a catalase. Various concentrations of catalase are tested (1 nM - 1 μM). Another secondary reaction aims at using $H_2O_2$ produced *in situ* as co-substrate of a peroxygenase reaction. In this order, to the photobiocatalytic CRO reaction is added a mixture containing an LPMO (1 μM), cellulose (10 $g.L^{-1}$ Avicel or 0.1% w/v phosphoric acid swollen cellulose) and a reducing agent (100 μM ascorbic acid or cellobiose (3 mM)/cellobiose dehydrogenase (CDH, 0.1-1 μM)). The LPMO is the AA9E from *Podospora anserina* (*Pa*LPMO9E) and the CDH is the CDH from *Podospora anserina* (*Pa*CDHB), produced and purified as previously described by C. Bennati Granier et al. "Substrate specificity and regioselectivity of fungal AA9 lytic polysaccharide monooxygenases secreted by Podospora anserina," Biotechnol. Biofuels, vol. 8, no. 1, p. 90, Dec. 2015.

**EXAMPLE 11: Photobioreactor upscaling**

After establishing the proof-of-concept in 1 mL reactions, the photobiocatalytic reactions is upscaled to 100 mL, using the same illumination system as described above but with a top-wise illumination, with the light source placed at optimal distance from the reaction mixture surface. The reaction is carried out in a 250 mL beaker without spout, under magnetic stirring, at 23 °C. The top of the beaker is closed with a home-made Quartz window, equipped with a rubber ring, to allow UV light to reach the solution while minimizing losses by evaporation.

**EXAMPLE 12: Reaction mixture analysis**

The CRO-AlcOx activity on benzyl alcohol is determined by quantifying benzaldehyde spectrophotometrically at 254 nm as described above.

The CRO-AlcOx activity on fatty alcohol is determined as follows: 500 μL of the reaction mixture is mixed with 500 μL of cyclohexane/ethyl acetate mixture (1:1), followed by shaking and centrifugation (5 min, 2300 *rpm*). The organic layer is transferred into a new vial by pipetting and injected in an Optima-σ-3 GC capillary column (30 m x 0.25 mm x 0.25 μm, Machery-Nagel GmbH&Co KG, Germany) mounted on a gas chromatography (GC)-2014 apparatus (Shimadzu, Japan) equipped with a flame ionization detector (FID). Nitrogen is used as carrier gas, under constant pressure (200 kPa). The inlet and detector temperature are set at 250 °C. The temperature gradients of the GC method are described in **Table 1.** Heptan-1-al (1 mM) is added as internal standard.

The CRO-GalOx activity on galactose is determined in two ways:

- For initial qualitative assessment of galactose consumption, we use thin layer chromatography (TLC), where the chromatograms are developed on silica gel plates (Sigma-Aldrich, L'Isle d'Abeau Chesnes, France) with butan-1-ol/acetic acid/$H_2O$ (4:1:1) as the solvent. The plates are dried before immersion in an acidic solution of orcinol (0.1% orcinol (w/v) dissolved in $H_2O$/ethanol/$H_2SO_4$ (22:75:3, vol/vol/vol) and visualized by heating at 105 °C for 5 min.

- For quantitation, we use high-performance anion exchange chromatography coupled to pulsed amperometric detection (HPAEC-PAD) (ICS-6000 system, ThermoFisher Scientific, Villebon sur Yvette, France). Samples are injected on a CarboPac PA1 (2 x 50 mm) column operated with 0.1M NaOH (eluent A) at a flow rate of 0.25 $ml.min^{-1}$ and a column temperature of 30 °C. Elution is achieved using a stepwise gradient with increasing amounts of eluent B (0.1 M NaOH, 1 M NaOAc), as follows: 0-10% B over 10 min; 10-30% B over 25 min; 30-100% B over 5 min; 100-0% B over 1 min; and 0% B (reconditioning) for 9 min. Chromatograms are recorded using Chromeleon 7.0 software.

The activities of CRO-GLOx on glyoxal/methylglyoxal/ glyoxylic acid and CRO-AAO on HMF are analyzed as previously described by M. Daou et al., "Heterologous production and characterization of two glyoxal oxidases from Pycnoporus cinnabarinus," Appl. Environ. Microbiol., vol. 82, no. 16, pp. 4867-4875, 2016. Briefly, the reaction products are separated on an Aminex HPX-87H column (300 x 7.8 mm; Bio-Rad) connected to a high pressure liquid chromatography (HPLC) apparatus (Agilent 1260) coupled to a diode-array detector (DAD, monitored wavelengths: 210, 280, 290, 330 and 350 nm). The column temperature is set to 45 °C, sulfuric acid (2.5 mM) is used as isocratic eluant with a flow rate of 0.5 $ml.min^{-1}$. All samples are filtered through 10-kDa-molecular-mass-cutoff Nanosep polyethersulfone membrane columns (Pall Corporation, Saint-Germainen-Laye, France) and 0.45-μm pore-size polyvinylidene difluoride syringe filters (Restek, Lisses, France) before injection in the column.

**Table 1. GC method**

| **Compounds** | **Rate** ($°C.min^{-1}$) | **T** (°C) | **Time** (min) |
|---|---|---|---|
| Hexanol | - | 40 | 4 |
| | 8 | 100 | 0 |
| | 25 | 220 | 3 |

## Claims

1. Use of UV-activated Copper Radical Oxidase (CRO) enzymes for the implementation of a chemical oxidation process of an organic compound,
in particular, wherein the chemical oxidation of the organic compound is followed by the formation of hydrogen peroxide.

2. A process for the chemical oxidation of an organic compound, said process comprising the step of contacting an organic compound bearing an oxidizable function with at least one Copper-Radical Oxidase (CRO) enzyme in the presence of molecular oxygen,
wherein said at least one CRO enzyme is activated by a step of exposing said at least one CRO enzyme to UV light, to obtain a UV-activated CRO enzyme,
whereby the organic compound is oxidized into an oxidized organic product,
and whereby hydrogen peroxide is generated.

3. The process according to claim 2, wherein the step of exposing said at least one CRO enzyme to UV light is carried out

    - during the step of contacting,
    - before the step of contacting, or
    - both before and during the step of contacting,

in particular wherein said step of exposing to UV light before the step of contacting is maintained for 1 second to about 10 minutes, followed by contacting said UV-activated enzyme with said organic compound.

**4.** The process for chemical oxidation according to claims 2 or 3,
wherein during the contact of said organic compound with said enzyme, the exposure to UV light is continuous or intermittent,
in particular, wherein during the contact of said organic compound with said enzyme, the exposure to UV light is intermittent, and preferably is maintained for one or more periods of 1 second to 1 hour, followed by one or more non-exposure periods of 1 second to 1 hour.

**5.** The process for chemical oxidation according to any one of claims 2 to 4, wherein the UV light has a wavelength comprised from 240 to 320 nm, preferably from 270 to 290 nm, more preferably has a wavelength of about 280 nm, in particular wherein, during the step of exposing said at least one CRO enzyme to UV light, the enzyme is exposed to UV light having a light intensity comprised from 0.01 to 1000 mW/cm$^2$, in particular from 1 to 100 mW/cm$^2$.

**6.** The process for chemical oxidation according to any one of claims 2 to 5, wherein said process comprises between the step exposing said at least one CRO enzyme to UV light before the step of contacting and the step of contacting said UV-activated enzyme with said organic compound,
a step of transfer whereby the organic compound and the UV-activated enzyme are mixed,
said step of transfer in particular being achieved in less than 10 minutes, preferably less than 1 minutes, and more preferably in less than 10 seconds.

**7.** The process for chemical oxidation according to any one of the claims 2 to 6,
wherein said process is carried out in an aqueous medium, preferably in a buffered aqueous medium, preferably at a temperature comprised between 20 and 50 °C preferably at a temperature of about 23 °C.

**8.** The process for chemical oxidation according to any of claims 2 to 7,
wherein the at least one CRO enzyme belongs to the AA5 family, in particular to the AA5_1 or the AA5_2 subfamilies.

**9.** The process for chemical oxidation according to anyone of claims 2 to 8,
wherein the at least one CRO enzyme belongs to the AA5_2 subfamily and is an alcohol oxidase (AlcOx), preferably is an alcohol oxidase extracted from *Colletotrichum graminicola,* in particular having SEQ *ID* NO: 1, or having at least 60%, in particular at least 70% identity with SEQ *ID* NO: 1,
or wherein the at least one CRO enzyme belongs to the AA5_2 subfamily and is a galactose oxidase (GalOx) and more preferably is a galactose oxidase extracted from *Fusarium graminearum,* in particular having SEQ *ID* NO: 2, or having at least 60%, in particular at least 70% identity with SEQ *ID* NO: 2.
or wherein the at least one CRO enzyme belongs to the AA5_2 subfamily and is an aryl alcohol oxidase (AAO) and is preferably extracted from *Colletotrichum graminicola,* in particular having SEQ *ID* NO: 3, or having at least 60%, in particular at least 70% identity with SEQ *ID* NO: 3.
or wherein the at least one CRO enzyme belongs to the AA5_1 subfamily and is a glyoxal oxidase (GLOx) and more preferably is a glyoxal oxidase extracted from *Pycnoporus cinnabarinus,* in particular having SEQ *ID* NO: 4, or having at least 60%, in particular at least 70% identity with SEQ *ID* NO: 4.
or wherein the at least one CRO enzyme is a GlxA-type enzyme which is preferably extracted from the bacterium *Streptomyces lividans,* in particular having SEQ *ID* NO: 5, or having at least 60%, in particular at least 70% identity with SEQ *ID* NO: 5.

**10.** The process for chemical oxidation according to any one of claims 2 to 9, wherein said organic compound is chosen from:

- saturated ($C_1$ to $C_{20}$) primary alcohols,
- unsaturated ($C_1$ to $C_{20}$) primary alcohols,
- saturated ($C_1$ to $C_{20}$) secondary alcohols,
- unsaturated ($C_1$ to $C_{20}$) secondary alcohols,
- ($C_3$ to $C_{10}$) cyclic alcohols,
- aryl alcohols,
- heteroaryl alcohols, and
- geminal diols,

in particular chosen from:

- saturated ($C_1$ to $C_{20}$) primary alcohols,
- allylic alcohols,
- aryl alcohols comprising a primary hydroxyl group linked to the aryl group by a $C_1$ alkyl group, and
- geminal diols.

**11.** Process for chemical oxidation according to any one of claims 2 to 10,
wherein said enzyme is an alcohol oxidase (AlcOx), and wherein said organic compound is:

- a primary alcohol and the obtained oxidized organic product is an aldehyde,
- an aryl alcohol comprising a primary hydroxyl group linked to the aryl group by a C1 alkyl group, in particular selected from benzyl alcohol, 4-nitrobenzyl alcohol, anisyl alcohol, veratryl alcohol and 4-hydroxybenzyl alcohol, or aryl alcohols comprising an allylic alcohol attached to the aryl group, in particular cinnamyl alcohol,
- a saturated, or unsaturated (C1 to C20) primary alcohol, linear or branched, in particular chosen from n-butanol, n-pentanol, n-hexanol or 2,4-hexadiene-1-ol, or
- a naturally-occurring polymer comprising long aliphatic chains bearing hydroxyl functions or a sugar, in particular polymers chosen from waxes, cutins and hemicellulose.

**12.** Process for chemical oxidation according to any one of claims 2 to 10, wherein said enzyme is a glyoxal oxidase (GLOx), and wherein said organic compound is:

- 5-hydroxymethylfurfuryl alcohol, or
- lignocellulose derived compounds, in particular glyoxal, methyl glyoxal, glyoxylic acid, formaldehyde or glycerol.

**13.** Process for chemical oxidation according to any one of claims 2 to 10, wherein said enzyme is a galactose oxidase (GalOx), and wherein said organic compound is:

- forest and agricultural biomass, in particular fibres, and hemicelluloses, in particular compounds comprising a galactopyranose moiety, more in particular xyloglucan.

**14.** Use of the hydrogen peroxide obtained by the process according to any one of claims 2 to 13, in hydrogen peroxide-driven enzymatic reactions.

**15.** Use of hydrogen peroxide according to claim 29, wherein the hydrogen peroxide-driven enzymatic reaction is chosen from decarboxylations, hydroxylations, halogenations, epoxidations, sulfoxidations and Baeyer-Villiger oxidations, or wherein the hydrogen peroxide-driven enzymatic reaction consists in the enzymatic conversion of said hydrogen peroxide into oxygen and water, in particular using a catalase enzyme,
or wherein the hydrogen peroxide-driven enzymatic reactions consists in the degradation of a polysaccharide, said reaction comprising contacting said polysaccharide with one or more lytic polysaccharide monooxygenase (LPMO), in the presence of an external source of electrons,
said source of electrons being in particular a reducing agent.

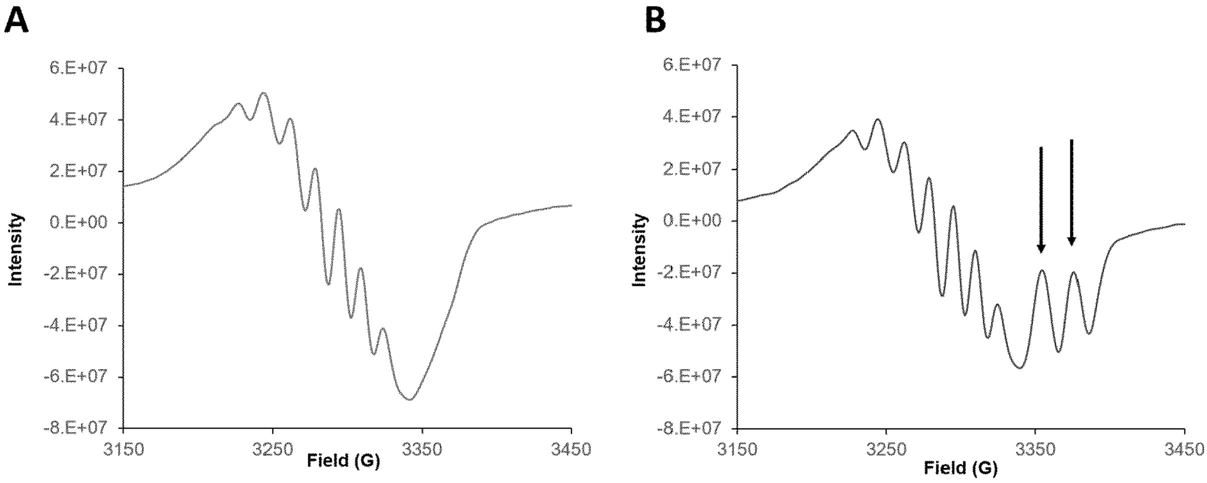


**Figure 1**

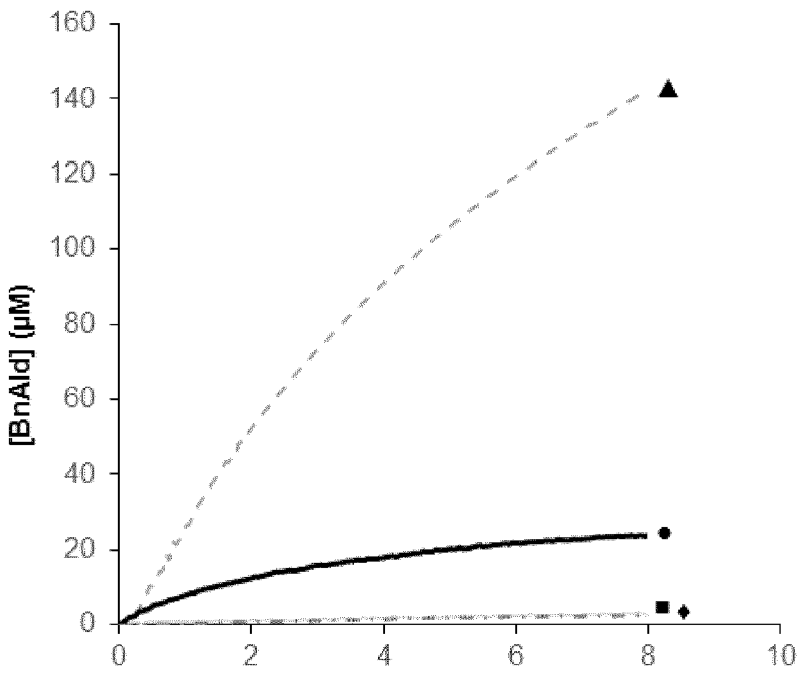


**Figure 2**

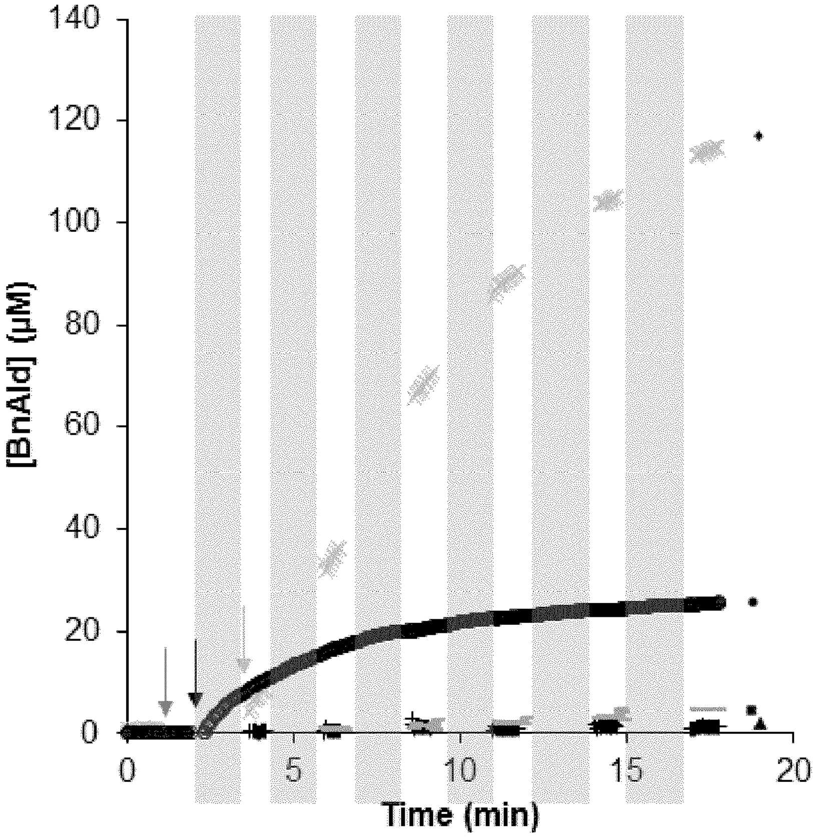


Figure 3

Europäisches Patentamt
European Patent Office
Office européen des brevets

# EUROPEAN SEARCH REPORT

Application Number
EP 20 18 6971

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y<br><br><br>A | US 2020/010869 A1 (MICKLITSCH CHRISTOPHER MICHAEL [US] ET AL) 9 January 2020 (2020-01-09)<br>* page 1, paragraph [0005]-[0006] *<br>* page 23, paragraph [0127] - page 24, paragraph [0128] *<br>* page 25, paragraph [0148] - page 26, paragraph [0151]; examples 1-31; sequences 4, 466,1048 *<br>----- | 1,2,14, 15<br><br><br>3-13 | INV.<br>C12N9/04<br>C12N9/02<br>C12P3/00<br>C12P7/02<br>C12P7/24 |
| Y<br><br>A | EP 3 599 281 A1 (UNIV AMSTERDAM [NL]) 29 January 2020 (2020-01-29)<br>* page 2, paragraph [0011] - page 4, paragraph [0030] *<br>* page 6, paragraph [0049]-[0051]; sequence 1 *<br>----- | 1,2,14, 15<br>3-13 | |
| A | EP 0 380 831 A1 (DU PONT [US]) 8 August 1990 (1990-08-08)<br>* column 1, lines 1-5 *<br>* column 2, lines 4-49; examples 1-4 *<br>* claims 1,8 *<br>----- | 1-13 | TECHNICAL FIELDS SEARCHED (IPC) |
| Y | KERSTEN PHIL ET AL: "Copper radical oxidases and related extracellular oxidoreductases of wood-decay Agaricomycetes", FUNGAL GENETICS AND BIOLOGY, SAN DIEGO, CA, US, vol. 72, 7 June 2014 (2014-06-07), pages 124-130, XP029084993, ISSN: 1087-1845, DOI: 10.1016/J.FGB.2014.05.011<br>* abstract *<br>* page 126, left-hand column, last paragraph - page 129, left-hand column, last paragraph *<br>-----<br>-/-- | 14,15 | C12N<br>C12P |

The present search report has been drawn up for all claims

| Place of search | Date of completion of the search | Examiner |
|---|---|---|
| Berlin | 14 January 2021 | Mateo Rosell, A |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 20 18 6971

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | DELU YIN ET AL: "Structure-function characterization reveals new catalytic diversity in the galactose oxidase and glyoxal oxidase family", NATURE COMMUNICATIONS, vol. 6, no. 1, 1 December 2015 (2015-12-01), XP055763604, DOI: 10.1038/ncomms10197 * page 2, left-hand column, paragraph 1 - page 9, left-hand column, paragraph 1; figures 1-5; table 1 * ----- | 1,2 | |
| Y | MARTÍNEZ ANGEL T ET AL: "Oxidoreductases on their way to industrial biotransformations", BIOTECHNOLOGY ADVANCES, ELSEVIER PUBLISHING, BARKING, GB, vol. 35, no. 6, 15 June 2017 (2017-06-15), pages 815-831, XP085179339, ISSN: 0734-9750, DOI: 10.1016/J.BIOTECHADV.2017.06.003 * abstract * * page 821, right-hand column, paragraph 2 - page 823, left-hand column, paragraph 1 * ----- | 1,2 | TECHNICAL FIELDS SEARCHED (IPC) |
| Y | B. O. BUREK ET AL: "Hydrogen peroxide driven biocatalysis", GREEN CHEMISTRY, vol. 21, no. 12, 1 January 2019 (2019-01-01), pages 3232-3249, XP055764794, ISSN: 1463-9262, DOI: 10.1039/C9GC00633H * abstract * * Schemes 1-19; page 3232, right-hand column, last paragraph - page 3240, left-hand column, paragraph 2 * ----- | 14,15 | |

The present search report has been drawn up for all claims

| Place of search | Date of completion of the search | Examiner |
|---|---|---|
| Berlin | 14 January 2021 | Mateo Rosell, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

page 2 of 2

## ANNEX TO THE EUROPEAN SEARCH REPORT ON EUROPEAN PATENT APPLICATION NO.

EP 20 18 6971

This annex lists the patent family members relating to the patent documents cited in the above-mentioned European search report. The members are as contained in the European Patent Office EDP file on The European Patent Office is in no way liable for these particulars which are merely given for the purpose of information.

14-01-2021

| Patent document cited in search report | Publication date | Patent family member(s) | Publication date |
|---|---|---|---|
| US 2020010869 A1 | 09-01-2020 | AU 2019300837 A1 | 07-01-2021 |
| | | CA 3103514 A1 | 16-01-2020 |
| | | US 2020010869 A1 | 09-01-2020 |
| | | WO 2020014049 A1 | 16-01-2020 |
| EP 3599281 A1 | 29-01-2020 | EP 3599281 A1 | 29-01-2020 |
| | | WO 2020020844 A1 | 30-01-2020 |
| EP 0380831 A1 | 08-08-1990 | EP 0380831 A1 | 08-08-1990 |
| | | JP H02215388 A | 28-08-1990 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ITO et al.** Novel Thioether Bond Revealed by a 1.7 A Crystal Structure of Galactose Oxidase. *Nature,* 07 March 1991, vol. 350 (6313), 87-90
- **FIRBANK et al.** Crystal structure of the precursor of galactose oxidase: An unusual self-processing enzyme. *Proc Natl Acad Sci USA.,* 06 November 2001, vol. 98 (23), 12932-12937
- **ROGER et al.** *J. Am. Chem. Soc.,* 2000, vol. 122 (5), 990-991
- **YIN et al.** Structure-function characterization reveals new catalytic diversity in the galactose oxidase and glyoxal oxidase family. *Nat. Commun.,* December 2015, vol. 6, 10197
- **O. SPADIUT et al.** A comparative summary of expression systems for the recombinant production of galactose oxidase. *Microb. Cell Fact.,* 2010, vol. 9, 1-13
- **M. DAOU et al.** Heterologous production and characterization of two glyoxal oxidases from Pycnoporus cinnabarinus. *Appl. Environ. Microbiol.,* 2016, vol. 82 (16), 4867-4875
- **Y. MATHIEU et al.** Discovery of a Fungal Copper Radical Oxidase with High Catalytic Efficiency toward 5-Hydroxymethylfurfural and Benzyl Alcohols for Bioprocessing. *ACS Catal.,* 2020, vol. 10 (5), 3042-3058
- **C. BENNATI GRANIER et al.** Substrate specificity and regioselectivity of fungal AA9 lytic polysaccharide monooxygenases secreted by Podospora anserina,. *Biotechnol. Biofuels,* December 2015, vol. 8 (1), 90
- **M. DAOU et al.** Heterologous production and characterization of two glyoxal oxidases from Pycnoporus cinnabarinus. *Appl. Environ. Microbiol,* 2016, vol. 82 (16), 4867-4875

SEQUENCE LISTING

<110> INSTITUT NATIONAL DE RECHERCHE POUR L'AGRICULTURE, L'ALIMENTATION ET L'ENVIRONNEMENT
CENTRE NATIONAL DE LA RECHERCHE SCIENTIFIQUE
AIX-MARSEILLE UNIVERSITE
ECOLE CENTRALE DE MARSEILLE

<120> USE OF UV-ACTIVATED ENZYMES TO IMPLEMENT OXIDATION REACTIONS AND THE CORRESPONDING PROCESSES

<130> IOB 20 BH INR CROX | B51667

<160> 5

<170> PatentIn version 3.5

<210> 1
<211> 506
<212> PRT
<213> Colletotrichum graminicola

<220>
<221> misc_feature
<223> Alcohol oxidase (AlcOx)

<400> 1

```
Met Pro Thr Leu Arg Ser Ala Leu Arg Asn Leu Pro Ala Ala Leu Leu
1               5                   10                  15

Ala Leu Ala Ala Ala Cys Glu Ala Gln Asn Val Gly Lys Trp Gly Pro
            20                  25                  30

Met Val Lys Phe Pro Val Val Pro Val Ala Val Ala Leu Val Pro Glu
        35                  40                  45

Thr Gly Asn Leu Leu Val Trp Ser Ser Gly Trp Pro Asn Arg Trp Thr
    50                  55                  60

Thr Ala Gly Asn Gly Lys Thr Tyr Thr Ser Leu Tyr Asn Val Asn Thr
65                  70                  75                  80

Gly Asn Ile Ser Asp Ala Ile Val Gln Asn Thr Gln His Asp Met Phe
                85                  90                  95

Cys Pro Gly Thr Ser Leu Asp Ala Asp Gly Arg Ile Ile Val Thr Gly
            100                 105                 110

Gly Ser Ser Ala Ala Lys Thr Ser Val Leu Asp Phe Lys Lys Gly Glu
        115                 120                 125

Ser Ser Pro Trp Thr Pro Leu Ser Asn Met Gln Ile Ser Arg Gly Tyr
    130                 135                 140
```

Gln Ser Ser Cys Thr Thr Ser Glu Gly Lys Ile Phe Val Ile Gly Gly
145 150 155 160

Ser Phe Ser Gly Ala Gly Thr Arg Asn Gly Glu Val Tyr Asp Pro Lys
165 170 175

Ala Asn Thr Trp Thr Lys Leu Ala Gly Cys Pro Val Lys Pro Leu Val
180 185 190

Met Gln Arg Gly Met Phe Pro Asp Ser His Ala Trp Leu Trp Ser Trp
195 200 205

Lys Asn Gly Ser Val Leu Gln Ala Gly Pro Ser Lys Lys Met Asn Trp
210 215 220

Tyr Asp Thr Lys Gly Thr Gly Ser Asn Thr Pro Ala Gly Leu Arg Gly
225 230 235 240

Thr Asp Glu Asp Ser Met Cys Gly Val Ser Val Met Tyr Asp Ala Val
245 250 255

Ala Gly Lys Ile Phe Thr Tyr Gly Gly Gly Lys Gly Tyr Thr Gly Tyr
260 265 270

Asp Ser Thr Ser Asn Ala His Ile Leu Thr Leu Gly Glu Pro Gly Gln
275 280 285

Ala Val Gln Val Gln Lys Leu Ala Asn Gly Lys Tyr Asn Arg Gly Phe
290 295 300

Ala Asn Ala Val Val Met Pro Asp Gly Lys Ile Trp Val Val Gly Gly
305 310 315 320

Met Gln Lys Met Trp Leu Phe Ser Asp Thr Thr Pro Gln Leu Thr Pro
325 330 335

Glu Leu Phe Asp Pro Ala Thr Gly Ser Phe Thr Pro Thr Thr Pro His
340 345 350

Thr Val Pro Arg Asn Tyr His Ser Thr Ala Leu Leu Met Ala Asp Ala
355 360 365

Thr Ile Trp Ser Gly Gly Gly Gly Leu Cys Gly Ala Asn Cys Lys Glu
370 375 380

Asn His Phe Asp Gly Gln Phe Trp Ser Pro Pro Tyr Leu Phe Glu Ala

385 390 395 400

Asp Gly Val Thr Pro Ala Lys Arg Pro Val Ile Gln Ser Leu Ser Asp
405 410 415

Thr Ala Val Arg Ala Gly Ala Pro Ile Thr Ile Thr Met Gln Asp Ala
420 425 430

Gly Ala Tyr Thr Phe Ser Met Ile Arg Val Ser Ala Thr Thr His Thr
435 440 445

Val Asn Thr Asp Gln Arg Arg Ile Pro Leu Asp Gly Gln Asp Gly Gly
450 455 460

Asp Gly Lys Ser Phe Thr Val Asn Val Pro Asn Asp Tyr Gly Val Ala
465 470 475 480

Ile Pro Gly Tyr Tyr Met Leu Phe Ala Met Asn Glu Ala Gly Val Pro
485 490 495

Cys Val Ala Gln Phe Phe Lys Val Thr Leu
500 505

<210> 2
<211> 680
<212> PRT
<213> Fusarium graminearum

<220>
<221> misc_feature
<223> Galactose oxidase (GalOx)

<400> 2

Met Lys His Leu Leu Thr Leu Ala Leu Cys Phe Ser Ser Ile Asn Ala
1 5 10 15

Val Ala Val Thr Val Pro His Lys Ala Val Gly Thr Gly Ile Pro Glu
20 25 30

Gly Ser Leu Gln Phe Leu Ser Leu Arg Ala Ser Ala Pro Ile Gly Ser
35 40 45

Ala Ile Ser Arg Asn Asn Trp Ala Val Thr Cys Asp Ser Ala Gln Ser
50 55 60

Gly Asn Glu Cys Asn Lys Ala Ile Asp Gly Asn Lys Asp Thr Phe Trp
65 70 75 80

```
His Thr Phe Tyr Gly Ala Asn Gly Asp Pro Lys Pro Pro His Thr Tyr
                85                  90                  95

Thr Ile Asp Met Lys Thr Thr Gln Asn Val Asn Gly Leu Ser Met Leu
            100                 105                 110

Pro Arg Gln Asp Gly Asn Gln Asn Gly Trp Ile Gly Arg His Glu Val
        115                 120                 125

Tyr Leu Ser Ser Asp Gly Thr Asn Trp Gly Ser Pro Val Ala Ser Gly
    130                 135                 140

Ser Trp Phe Ala Asp Ser Thr Thr Lys Tyr Ser Asn Phe Glu Thr Arg
145                 150                 155                 160

Pro Ala Arg Tyr Val Arg Leu Val Ala Ile Thr Glu Ala Asn Gly Gln
                165                 170                 175

Pro Trp Thr Ser Ile Ala Glu Ile Asn Val Phe Gln Ala Ser Ser Tyr
            180                 185                 190

Thr Ala Pro Gln Pro Gly Leu Gly Arg Trp Gly Pro Thr Ile Asp Leu
        195                 200                 205

Pro Ile Val Pro Ala Ala Ala Ala Ile Glu Pro Thr Ser Gly Arg Val
    210                 215                 220

Leu Met Trp Ser Ser Tyr Arg Asn Asp Ala Phe Gly Gly Ser Pro Gly
225                 230                 235                 240

Gly Ile Thr Leu Thr Ser Ser Trp Asp Pro Ser Thr Gly Ile Val Ser
                245                 250                 255

Asp Arg Thr Val Thr Val Thr Lys His Asp Met Phe Cys Pro Gly Ile
            260                 265                 270

Ser Met Asp Gly Asn Gly Gln Ile Val Val Thr Gly Gly Asn Asp Ala
        275                 280                 285

Lys Lys Thr Ser Leu Tyr Asp Ser Ser Ser Asp Ser Trp Ile Pro Gly
    290                 295                 300

Pro Asp Met Gln Val Ala Arg Gly Tyr Gln Ser Ser Ala Thr Met Ser
305                 310                 315                 320

Asp Gly Arg Val Phe Thr Ile Gly Gly Ser Trp Ser Gly Gly Val Phe
                325                 330                 335
```

```
Glu Lys Asn Gly Glu Val Tyr Ser Pro Ser Ser Lys Thr Trp Thr Ser
            340                 345                 350

Leu Pro Asn Ala Lys Val Asn Pro Met Leu Thr Ala Asp Lys Gln Gly
        355                 360                 365

Leu Tyr Arg Ser Asp Asn His Ala Trp Leu Phe Gly Trp Lys Lys Gly
    370                 375                 380

Ser Val Phe Gln Ala Gly Pro Ser Thr Ala Met Asn Trp Tyr Tyr Thr
385                 390                 395                 400

Ser Gly Ser Gly Asp Val Lys Ser Ala Gly Lys Arg Gln Ser Asn Arg
                405                 410                 415

Gly Val Ala Pro Asp Ala Met Cys Gly Asn Ala Val Met Tyr Asp Ala
            420                 425                 430

Val Lys Gly Lys Ile Leu Thr Phe Gly Gly Ser Pro Asp Tyr Gln Asp
        435                 440                 445

Ser Asp Ala Thr Thr Asn Ala His Ile Ile Thr Leu Gly Glu Pro Gly
    450                 455                 460

Thr Ser Pro Asn Thr Val Phe Ala Ser Asn Gly Leu Tyr Phe Ala Arg
465                 470                 475                 480

Thr Phe His Thr Ser Val Val Leu Pro Asp Gly Ser Thr Phe Ile Thr
                485                 490                 495

Gly Gly Gln Arg Arg Gly Ile Pro Phe Glu Asp Ser Thr Pro Val Phe
            500                 505                 510

Thr Pro Glu Ile Tyr Val Pro Glu Gln Asp Thr Phe Tyr Lys Gln Asn
        515                 520                 525

Pro Asn Ser Ile Val Arg Val Tyr His Ser Ile Ser Leu Leu Leu Pro
    530                 535                 540

Asp Gly Arg Val Phe Asn Gly Gly Gly Gly Leu Cys Gly Asp Cys Thr
545                 550                 555                 560

Thr Asn His Phe Asp Ala Gln Ile Phe Thr Pro Asn Tyr Leu Tyr Asn
                565                 570                 575

Ser Asn Gly Asn Leu Ala Thr Arg Pro Lys Ile Thr Arg Thr Ser Thr
            580                 585                 590
```

Gln Ser Val Lys Val Gly Gly Arg Ile Thr Ile Ser Thr Asp Ser Ser
595 600 605

Ile Ser Lys Ala Ser Leu Ile Arg Tyr Gly Thr Ala Thr His Thr Val
610 615 620

Asn Thr Asp Gln Arg Arg Ile Pro Leu Thr Leu Thr Asn Asn Gly Gly
625 630 635 640

Asn Ser Tyr Ser Phe Gln Val Pro Ser Asp Ser Gly Val Ala Leu Pro
645 650 655

Gly Tyr Trp Met Leu Phe Val Met Asn Ser Ala Gly Val Pro Ser Val
660 665 670

Ala Ser Thr Ile Arg Val Thr Gln
675 680

<210> 3
<211> 711
<212> PRT
<213> Colletotrichum graminicola

<220>
<221> misc_feature
<223> Aryl alcohol oxidase (AAO)

<400> 3

Met Val Arg Ser Cys Ala Tyr Lys Ala Ile Ala Ala Ala Ser Leu Leu
1 5 10 15

Ser Gln Leu Ala Ser Ala Ala Ile Thr Ser Cys Pro Asn Asn Glu Thr
20 25 30

Val Trp Glu Thr Pro Ile Gly Val Lys Tyr Thr Leu Cys Pro Gly Ser
35 40 45

Asp Tyr Gln Asn Gly Gly Ala Ser Leu Gln Thr Val Arg Asp Ile Gln
50 55 60

Ser Ser Leu Glu Cys Ala Lys Ile Cys Asp Ser Asp Ala Arg Cys Asn
65 70 75 80

Arg Ala Val Tyr Asp Asn Val Asn Lys Ala Cys Asp Val Lys Asn Ser
85 90 95

Thr Asn Pro Met Gln Trp Ala Ala Asp Asp Arg Phe Glu Thr Ile Arg

```
            100                 105                 110

Leu Thr Asn Asp Leu Pro Glu Gly Ala Phe Ile Ser Thr Cys Ser Phe
        115                 120                 125

Asn Glu Thr Ser Tyr Arg Val Pro Glu Thr Asn Ala Glu Tyr Arg Ile
    130                 135                 140

Cys Pro Asp Thr Asp Tyr Thr Gly Val Asn Ala Lys Val Val Glu Gly
145                 150                 155                 160

Val Thr Thr Ile Gln Ala Cys Ala Glu Leu Cys Ser Asn Thr Gln Asp
                165                 170                 175

Cys Arg Lys Ser Val Phe Asp His Ile Asn Asn Ala Cys Ala Ile Lys
            180                 185                 190

Ala Ala Glu Pro Ala Thr Ser Ile Phe Trp Val Gln Asp Lys Gln Phe
        195                 200                 205

Ser Thr Ile Arg Leu Pro Glu Asn Ile Asp Pro Ala Val Lys Gly Lys
    210                 215                 220

Trp Gly Asp Leu Ile Arg Leu Pro Val Ile Pro Val Ala Ala Tyr Ile
225                 230                 235                 240

Val Pro Ser Tyr Pro Glu Pro Ser Arg Leu Leu Phe Phe Ser Ser Trp
                245                 250                 255

Ser Asn Asp Ala Phe Ser Gly Ala Ser Gly Met Thr Gln Phe Gly Asp
            260                 265                 270

Tyr Asp Phe Ala Thr Gly Ala Ile Ser Gln Arg Thr Val Thr Asn Thr
        275                 280                 285

His His Asp Met Phe Cys Pro Gly Ile Ser Gln Leu Glu Asp Gly Arg
    290                 295                 300

Ile Leu Ile Gln Gly Gly Ser Asp Ala Asp Thr Val Ser Ile Tyr Asp
305                 310                 315                 320

Pro Ala Thr Asn Glu Phe Thr Arg Gly Pro Asn Met Thr Leu Ala Arg
                325                 330                 335

Gly Tyr Gln Thr Ser Cys Thr Leu Ser Asn Gly Lys Val Phe Thr Ile
            340                 345                 350
```

```
Gly Gly Ala Tyr Ser Gly Glu Arg Val Gly Lys Asn Gly Glu Val Tyr
        355                 360                 365

Asp Pro Val Ala Asn Ala Trp Thr Tyr Leu Pro Gly Ala Asp Phe Arg
    370                 375                 380

Pro Met Leu Thr Asn Asp His Glu Gly Ile Trp Arg Glu Asp Asn His
385                 390                 395                 400

Ala Trp Leu Phe Gly Trp Lys Asn Gly Ser Ile Phe Gln Ala Gly Pro
                405                 410                 415

Ser Lys Asp Gln His Trp Tyr Gly Ile Gln Gly Asn Gly Thr Val Ala
            420                 425                 430

Lys Ala Ala Thr Arg Asp Asp Asp Asp Ala Met Cys Gly Val Trp Val
        435                 440                 445

Met Tyr Asp Ala Val Ala Gly Lys Ile Phe Ser Ala Gly Gly Ser Pro
    450                 455                 460

Asp Tyr Thr Asp Ser Pro Ala Thr Gln Arg Ala His Ile Thr Thr Ile
465                 470                 475                 480

Gly Glu Pro Asn Thr Pro Ala Glu Val Glu Arg Val Ala Asp Met Gly
                485                 490                 495

Phe Pro Arg Gly Phe Ala Asn Ala Val Val Leu Pro Asp Gly Gln Val
            500                 505                 510

Leu Val Thr Gly Gly Gln Arg Met Ser Leu Val Phe Thr Asn Thr Asp
        515                 520                 525

Gly Ile Leu Val Ala Glu Leu Phe Asn Pro Glu Thr Arg Glu Trp Lys
    530                 535                 540

Gln Met Ala Pro Met Ala Val Pro Arg Asn Tyr His Ser Val Ser Ile
545                 550                 555                 560

Leu Leu Pro Asp Ala Thr Val Phe Ser Gly Gly Gly Gly Met Cys Trp
                565                 570                 575

Val Gln Asn Val Gly Asp Ser Thr Ala Gly Cys Asp Lys Thr Val Asp
            580                 585                 590

His Ser Asp Gly Glu Ile Phe Glu Pro Pro Tyr Leu Phe Asn Glu Asp
        595                 600                 605
```

Gly Ser Arg Ala Ala Arg Pro Val Ile Ser Ala Ile Ser Ala Asp Pro
610 615 620

Ile Lys Ala Gly Ala Thr Leu Thr Phe Thr Val Glu Gly Val Glu Gly
625 630 635 640

Gln Gly Thr Ala Ala Leu Ile Arg Leu Gly Ser Val Thr His Ser Val
645 650 655

Asn Ser Asp Gln Arg Arg Val Pro Leu Asn Val Thr Val Ser Gly Asn
660 665 670

Glu Tyr Ser Ala Thr Leu Pro Asp Asp Tyr Gly Ile Leu Leu Pro Gly
675 680 685

Tyr Tyr Tyr Leu Phe Val Ser Thr Pro Gln Gly Thr Pro Ser Ile Ala
690 695 700

Lys Thr Val His Val Ile Leu
705 710

<210> 4
<211> 559
<212> PRT
<213> Pycnoporus cinnabarinus

<220>
<221> misc_feature
<223> Glyoxal oxidase (GLOx)

<400> 4

Met Phe Gln Thr Thr Leu His Leu Leu Phe Val Leu Val Val Thr Gly
1 5 10 15

Arg Gly Leu Ala Ala Pro Ser Thr Pro Thr Gly Trp Gln Phe Asn Leu
20 25 30

Lys Ala Glu Arg Ser Gly Ile Val Ala Leu Glu Ser Ile Val Val Ser
35 40 45

Pro Thr Leu Val Val Phe Phe Asp Arg Ala Thr Asn Asp Pro Leu Gln
50 55 60

Ile Asn Asn His Ser Ala Trp Gly Ala Leu Trp Asn Leu Glu Thr Ser
65 70 75 80

Thr Val Arg Ala Leu Asp Val Leu Thr Asn Ser Phe Cys Ala Ser Gly
85 90 95

Ala Leu Leu Ser Asn Gly Thr Met Ala Ser Ile Gly Gly Asp Pro Asn
100 105 110

Gly Phe Pro Gly Asn Pro Ala Ile His Pro Gly Thr Gln Ala Ile Arg
115 120 125

Leu Phe Glu Pro Cys Asp Ser Pro Thr Gly Glu Gly Cys Thr Leu Phe
130 135 140

Glu Asp Pro Val Thr Leu His Leu Leu Glu Lys Arg Trp Tyr Pro Ser
145 150 155 160

Ser Val Arg Ile Phe Asp Gly Ser Leu Leu Ile Val Gly Gly Met His
165 170 175

Glu Glu Thr Pro Phe Tyr Asn Thr Asp Pro Ala Leu Ser Phe Glu Phe
180 185 190

Phe Pro Pro Lys Glu Ser Thr Pro Arg Pro Ser Glu Phe Leu Asn Arg
195 200 205

Ser Leu Pro Ala Asn Leu Phe Pro Arg Val Phe Ala Leu Pro Asp Gly
210 215 220

Lys Val Phe Met Val Ala Asn Asn Gln Ser Ile Ile Tyr Asp Ile Glu
225 230 235 240

Ala Asn Thr Glu Arg Ile Leu Pro Asp Ile Pro Asn Asn Val Arg Val
245 250 255

Thr Asn Pro Ile Asp Gly Ser Ala Ile Leu Leu Pro Leu Ser Pro Pro
260 265 270

Asp Phe Val Pro Glu Val Leu Val Cys Gly Gly Thr Gln Thr Asp Thr
275 280 285

Ile Asp Pro Ser Leu Leu Thr Ser Gln Thr Pro Ala Ser Ser Gln Cys
290 295 300

Ser Arg Ile Arg Leu Asp Glu Glu Gly Ile Ala Arg Gly Trp Glu Val
305 310 315 320

Glu His Met Leu Glu Gly Arg Met Met Pro Glu Leu Val His Leu Pro
325 330 335

Asn Gly Gln Val Leu Ile Ala Asn Gly Ala Arg Thr Gly Phe Ala Ala

```
            340                 345                 350

Ile Ala Ser Val Ser Asp Pro Val Gly Gly Ser Asn Ala Asp His Ala
        355                 360                 365

Val Leu Val Pro Ser Leu Tyr Thr Pro Asp Ala Pro Leu Gly Thr Arg
    370                 375                 380

Ile Ser Asn Val Gly Leu Pro Ser Ser Gly Ile Ala Arg Val Tyr His
385                 390                 395                 400

Ser Ser Ile Thr Leu Thr Pro Gln Gly Asn Phe Leu Ile Ala Gly Ser
                405                 410                 415

Asn Pro Asn Asn Asn Ser Ser Val Thr Ala Gly Val Lys Phe Pro Ser
            420                 425                 430

Glu Phe Arg Val Gln Thr Leu Asp Pro Pro Phe Met Phe Val Glu Arg
        435                 440                 445

Pro Lys Ile Leu Ser Met Pro Lys Lys Leu Ala Phe Gly Lys Ser Phe
    450                 455                 460

Thr Val Pro Ile Ala Val Pro Ser Thr Leu Ala His Pro Gly Ala Lys
465                 470                 475                 480

Val Gln Val Ser Leu Met Asp Leu Gly Phe Ser Ser His Ala Phe His
                485                 490                 495

Ser Ser Ala Arg Leu Val Phe Met Asn Ala Lys Ile Ser Gln Asp Gly
            500                 505                 510

Lys Ser Leu Thr Phe Thr Thr Pro Pro Asn Gly Arg Val Tyr Pro Pro
        515                 520                 525

Gly Pro Ala Thr Ile Phe Leu Thr Ile Asp Asp Val Thr Ser Glu Gly
    530                 535                 540

Ala Trp Val Met Met Gly Ser Gly Asn Pro Pro Pro Thr Leu Glu
545                 550                 555
```

<210>  5
<211>  644
<212>  PRT
<213>  Streptomyces lividans

<220>
<221>  misc_feature

<223> GlxA-type enzyme

<400> 5

Met Lys Asp Arg Ala Gly Arg Arg Arg Ala Arg Arg Phe Ala Ile Gly
1 5 10 15

Thr Ala Val Val Val Ala Leu Ala Gly Met Asn Gly Pro Trp Leu Tyr
20 25 30

Arg Phe Ser Thr Glu Lys Tyr His Gln Tyr Lys Ile Asn Gln Pro Glu
35 40 45

Tyr Lys Ala Ala Asn Gly Lys Trp Glu Ile Ile Glu Phe Pro Glu Lys
50 55 60

Tyr Arg Gln Asn Thr Ile His Ala Ala Leu Leu Arg Thr Gly Lys Val
65 70 75 80

Leu Met Val Ala Gly Ser Gly Asn Asn Gln Asp Asn Ser Asp Asp Lys
85 90 95

Gln Tyr Asp Thr Arg Ile Trp Asp Pro Val Lys Gly Thr Ile Lys Lys
100 105 110

Val Pro Thr Pro Ser Asp Leu Phe Cys Thr Gly His Thr Gln Leu Ala
115 120 125

Asn Gly Asn Leu Leu Ile Ala Gly Gly Thr Lys Arg Tyr Glu Lys Leu
130 135 140

Lys Gly Asp Val Thr Lys Ala Gly Gly Leu Met Val Val His Asn Glu
145 150 155 160

Asn Pro Asp Lys Pro Ile Thr Leu Pro Ala Gly Thr Lys Phe Thr Gly
165 170 175

Lys Glu Asn Gly Lys Thr Phe Val Ser Lys Asp Pro Val Leu Val Pro
180 185 190

Arg Ala Glu Lys Val Phe Asp Pro Ala Thr Gly Ala Phe Val Arg Asn
195 200 205

Asp Pro Gly Leu Gly Arg Ile Tyr Val Glu Ala Gln Lys Ser Gly Ser
210 215 220

Ala Tyr Glu Thr Gly Thr Glu Asp Asn Tyr Arg Val Gln Gly Leu Ser
225 230 235 240

```
Gly Ala Asp Ala Arg Asn Thr Tyr Gly Ile Ala Gln Lys Leu Ala Leu
                245                 250                 255

Asp Lys Lys Asp Phe Gln Gly Ile Arg Asp Ala Phe Glu Phe Asp Pro
            260                 265                 270

Val Ala Glu Lys Tyr Ile Lys Val Asp Pro Met His Glu Ala Arg Trp
        275                 280                 285

Tyr Pro Thr Leu Thr Thr Leu Gly Asp Gly Lys Leu Ser Val Ser Gly
    290                 295                 300

Leu Asp Asp Ile Gly Gln Leu Val Pro Gly Lys Asn Glu Val Tyr Asp
305                 310                 315                 320

Pro Lys Thr Lys Ala Trp Thr Tyr Thr Asp Lys Val Arg Gln Phe Pro
                325                 330                 335

Thr Tyr Pro Ala Leu Phe Leu Met Gln Asn Gly Lys Ile Phe Tyr Ser
            340                 345                 350

Gly Ala Asn Ala Gly Tyr Gly Pro Asp Asp Val Gly Arg Thr Pro Gly
        355                 360                 365

Val Trp Asp Val Glu Thr Asn Lys Phe Thr Lys Val Pro Gly Met Ser
    370                 375                 380

Asp Ala Asn Met Leu Glu Thr Ala Asn Thr Val Leu Leu Pro Pro Ala
385                 390                 395                 400

Gln Asp Glu Lys Tyr Met Val Ile Gly Gly Gly Gly Val Gly Glu Ser
                405                 410                 415

Lys Leu Ser Ser Glu Lys Thr Arg Ile Ala Asp Leu Lys Ala Asp Asp
            420                 425                 430

Pro Lys Phe Val Asp Gly Pro Ser Leu Glu Lys Gly Thr Arg Tyr Pro
        435                 440                 445

Gln Ala Ser Ile Leu Pro Asp Asp Ser Val Leu Val Ser Gly Gly Ser
    450                 455                 460

Gln Asp Tyr Arg Gly Arg Gly Asp Ser Asn Ile Leu Gln Ala Arg Leu
465                 470                 475                 480

Tyr His Pro Asp Thr Asn Glu Phe Glu Arg Val Ala Asp Pro Leu Val
                485                 490                 495
```

```
Gly Arg Asn Tyr His Ser Gly Ser Ile Leu Leu Pro Asp Gly Arg Leu
            500                 505                 510

Met Phe Phe Gly Ser Asp Ser Leu Tyr Ala Asp Lys Ala Asn Thr Lys
        515                 520                 525

Pro Gly Lys Phe Glu Gln Arg Ile Glu Ile Tyr Thr Pro Pro Tyr Leu
    530                 535                 540

Tyr Arg Asp Ser Arg Pro Asp Leu Ser Gly Gly Pro Gln Thr Ile Ala
545                 550                 555                 560

Arg Gly Gly Ser Gly Thr Phe Thr Ser Arg Ala Ala Ser Thr Val Lys
                565                 570                 575

Lys Val Arg Leu Ile Arg Pro Ser Ala Ser Thr His Val Thr Asp Val
            580                 585                 590

Asp Gln Arg Ser Ile Ala Leu Asp Phe Lys Ala Asp Gly Asp Lys Leu
        595                 600                 605

Thr Val Thr Val Pro Ser Gly Lys Asn Leu Val Gln Ser Gly Trp Tyr
    610                 615                 620

Met Met Phe Val Thr Asp Gly Glu Gly Thr Pro Ser Lys Ala Glu Trp
625                 630                 635                 640

Val Arg Val Pro
```